(19) Europäisches Patentamt
European Patent Office
Office européen des brevets

(11) EP 1 689 741 B1

(12) EUROPEAN PATENT SPECIFICATION

(45) Date of publication and mention of the grant of the patent:
**13.02.2008 Bulletin 2008/07**

(21) Application number: **04798661.7**

(22) Date of filing: **25.11.2004**

(51) Int Cl.:
*C07D 405/12* (2006.01) *A61K 31/443* (2006.01)
*A61K 31/445* (2006.01) *A61P 3/10* (2006.01)

(86) International application number:
**PCT/GB2004/004956**

(87) International publication number:
**WO 2005/054233 (16.06.2005 Gazette 2005/24)**

(54) **BENZOYL AMINO PYRIDYL CARBOXYLIC ACID DERIVATIVES AS GLUCOKINASE ACTIVATORS**

BENZOYLAMINOPYRIDYLCARBONSÄUREDERIVATE ALS GLUCOKINASEAKTIVATOREN

DERIVES D'ACIDE CARBOXYLIQUE BENZOYL-AMINO-PYRIDILES UTILISES COMME ACTIVATEURS DE GLUCOKINASE

(84) Designated Contracting States:
**AT BE BG CH CY CZ DE DK EE ES FI FR GB GR HU IE IS IT LI LU MC NL PL PT RO SE SI SK TR**

(30) Priority: **29.11.2003 GB 0327760**

(43) Date of publication of application:
**16.08.2006 Bulletin 2006/33**

(73) Proprietor: **AstraZeneca AB**
**151 85 Södertälje (SE)**

(72) Inventors:
- **JOHNSTONE, Craig**
**Macclesfield**
**Cheshire SK10 4TG (GB)**
- **MCKERRECHER, Darren**
**Macclesfield**
**Cheshire SK10 4TG (GB)**
- **PIKE, Kurt, Gordon**
**Macclesfield**
**Cheshire SK10 4TG (GB)**

(56) References cited:
**WO-A-03/000267**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 689 741 B1

## Description

The present invention relates to a group of benzoyl amino pyridyl carboxylic acids which are useful in the treatment or prevention of a disease or medical condition mediated through glucokinase (GLK), leading to a decreased glucose threshold for insulin secretion. In addition the compounds are predicted to lower blood glucose by increasing hepatic glucose uptake. Such compounds may have utility in the treatment of Type 2 diabetes and obesity. The invention also relates to pharmaceutical compositions comprising said compounds and to methods of treatment of diseases mediated by GLK using said compounds.

In the pancreatic β-cell and liver parenchymal cells the main plasma membrane glucose transporter is GLUT2. Under physiological glucose concentrations the rate at which GLUT2 transports glucose across the membrane is not rate limiting to the overall rate of glucose uptake in these cells. The rate of glucose uptake is limited by the rate of phosphorylation of glucose to glucose-6-phosphate (G-6-P) which is catalysed by glucokinase (GLK) [1]. GLK has a high (6-10mM) Km for glucose and is not inhibited by physiological concentrations of G-6-P [1]. GLK expression is limited to a few tissues and cell types, most notably pancreatic β-cells and liver cells (hepatocytes) [1]. In these cells GLK activity is rate limiting for glucose utilisation and therefore regulates the extent of glucose induced insulin secretion and hepatic glycogen synthesis. These processes are critical in the maintenance of whole body glucose homeostasis and both are dysfunctional in diabetes [2].

In one sub-type of diabetes, Type 2 maturity-onset diabetes of the young (MODY-2), the diabetes is caused by GLK loss of function mutations [3,4]. Hyperglycaemia in MODY-2 patients results from defective glucose utilisation in both the pancreas and liver [5]. Defective glucose utilisation in the pancreas of MODY-2 patients results in a raised threshold for glucose stimulated insulin secretion. Conversely, rare activating mutations of GLK reduce this threshold resulting in familial hyperinsulinism [6, 6a, 7]. In addition to the reduced GLK activity observed in MODY-2 diabetics, hepatic glucokinase activity is also decreased in type 2 diabetics [8]. Importantly, global or liver selective overexpression of GLK prevents or reverses the development of the diabetic phenotype in both dietary and genetic models of the disease [9-12]. Moreover, acute treatment of type 2 diabetics with fructose improves glucose tolerance through stimulation of hepatic glucose utilisation [13]. This effect is believed to be mediated through a fructose induced increase in cytosolic GLK activity in the hepatocyte by the mechanism described below [13].

Hepatic GLK activity is inhibited through association with GLK regulatory protein (GLKRP). The GLK/GLKRP complex is stabilised by fructose-6-phosphate (F6P) binding to the GLKRP and destabilised by displacement of this sugar phosphate by fructose-1-phosphate (F1P). F1P is generated by fructokinase mediated phosphorylation of dietary fructose. Consequently, GLK/GLKRP complex integrity and hepatic GLK activity is regulated in a nutritionally dependent manner as F6P is elevated in the post-absorptive state whereas F1P predominates in the post-prandial state. In contrast to the hepatocyte, the pancreatic β-cell expresses GLK in the absence of GLKRP. Therefore, β-cell GLK activity is regulated exclusively by the availability of its substrate, glucose. Small molecules may activate GLK either directly or through destabilising the GLK/GLKRP complex. The former class of compounds are predicted to stimulate glucose utilisation in both the liver and the pancreas whereas the latter are predicted to act exclusively in the liver. However, compounds with either profile are predicted to be of therapeutic benefit in treating Type 2 diabetes as this disease is characterised by defective glucose utilisation in both tissues.

GLK and GLKRP and the $K_{ATP}$ channel are expressed in neurones of the hypothalamus, a region of the brain that is important in the regulation of energy balance and the control of food intake [14-18]. These neurones have been shown to express orectic and anorectic neuropeptides [15, 19, 20] and have been assumed to be the glucose-sensing neurones within the hypothalamus that are either inhibited or excited by changes in ambient glucose concentrations [17, 19, 21, 22]. The ability of these neurones to sense changes in glucose levels is defective in a variety of genetic and experimentally induced models of obesity [23-28]. Intracerebroventricular (icv) infusion of glucose analogues, that are competitive inhibitors of glucokinase, stimulate food intake in lean rats [29, 30]. In contrast, icv infusion of glucose suppresses feeding [31]. Thus, small molecule activators of GLK may decrease food intake and weight gain through central effects on GLK. Therefore, GLK activators may be of therapeutic use in treating eating disorders, including obesity, in addition to diabetes. The hypothalamic effects will be additive or synergistic to the effects of the same compounds acting in the liver and/or pancreas in normalising glucose homeostasis, for the treatment of Type 2 diabetes. Thus the GLK/GLKRP system can be described as a potential "Diabesity" target (of benefit in both Diabetes and Obesity).

In WO0058293 and WO01/44216 (Roche), a series of benzylcarbamoyl compounds are described as glucokinase activators. The mechanism by which such compounds activate GLK is assessed by measuring the direct effect of such compounds in an assay in which GLK activity is linked to NADH production, which in turn is measured optically - see details of the *in vitro* assay described in Example A. Compounds of the present invention may activate GLK directly or may activate GLK by inhibiting the interaction of GLKRP with GLK. The latter mechanism offers an important advantage over direct activators of GLK in that they will not cause the severe hypoglycaemic episodes predicted after direct stimulation. Many compounds of the present invention may show favourable selectivity compared to known GLK activators.

WO9622282, WO9622293, WO9622294, WO9622295, WO9749707 and WO9749708 disclose a number of

intermediates used in the preparation of compounds useful as vasopressin agents which are structurally similar to those disclosed in the present invention. Structurally similar compounds are also disclosed in WO9641795 and JP8143565 (vasopressin antagonism), in JP8301760 (skin damage prevention) and in EP619116 (osetopathy).

WO01/12621 describes the preparation of as isoxazolylpyrimidines and related compounds as inhibitors of c-JUN N-terminal kinases, and pharmaceutical compositions containing such compounds.

Cushman et al [Bioorg Med Chem Lett (1991) 1(4), 211-14] describe the synthesis of pyridine-containing stilbenes and amides and their evaluation as protein-tyrosine kinase inhibitors. Rogers et al [J Med Chem (1981) 24(11) 1284-7] describe mesoionic purinone analogs as inhibitors of cyclic-AMP phosphodiesterase.

WO00/26202 describes the preparation of 2-amino-thiazole derivatives as antitumour agents. GB 2331748 describes the preparation of insecticidal thiazole derivatives. WO96/36619 describes the preparation of aminothiazole derivatives as ameliorating agents for digestive tract movements. US 5466715 and US 5258407 describe the preparation of 3,4-disubstituted phenol immunostimulants. JP 58069812 describes hypoglycemic pharmaceuticals containing benzamide derivatives. US 3950351 describes 2-benzamido-5-nitrothiazoles and Cavier et al [Eur J Med Chem - Chim Ther (1978) 13(6), 539-43] discuss the biological interest of these compounds.

WO03/000262 discloses vinylphenyl derivatives as GLK activators, WO03/015774 discloses benzamide compounds as GLK activators and WO03/066613 discloses N-phenyl-2-pyrimidinamine derivatives as GLK activators.

Pending International application Number: PCT/GB02/02873 (WO03/000267) describes a group of benzoyl amino pyridyl carboxylic acids which are activators of the enzyme glucokinase (GLK). We have surprisingly found a small selection of these compounds which have a superior level of drug in plasma following oral administration which is due to improved aqueous solubility and decreased levels of plasma binding, whilst retaining high potency for the GLK enzyme. This makes this sub-group of compounds particularly suitable for use in the treatment or prevention of a disease or medical condition mediated through GLK.

Thus, according to the first aspect of the invention there is provided a compound of Formula (I):

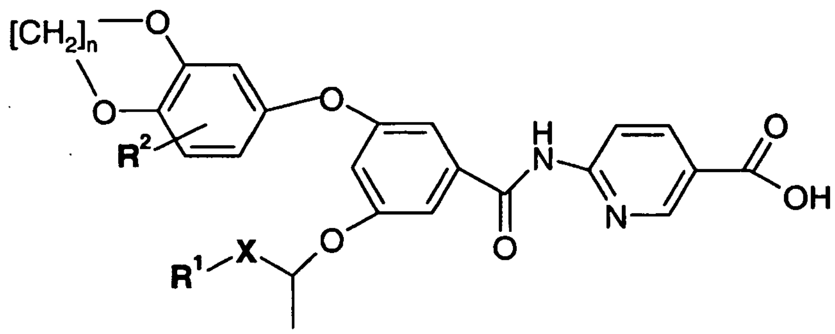

Formula (I)

wherein:

**$R^1$-X-** is selected from: methyl, methoxymethyl and

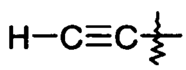

;

**$R^2$** is selected from hydrogen, methyl, chloro and fluoro;
**n** is 1 or 2;

or a salt, in-vivo hydrolysable ester or solvate thereof

For the avoidance of doubt $R^2$ is a single group which can be substituted on either of the 2, 3 or 6 positions relative to the oxygen atom between the two phenyl rings.

Compounds of Formula (I) may form salts which are within the ambit of the invention. Pharmaceutically acceptable salts are preferred although other salts may be useful in, for example, isolating or purifying compounds.

It is to be understood that, insofar as certain of the compounds of Formula (I) defined above may exist in optically active or racemic forms by virtue of one or more asymmetric carbon atoms, the invention includes in its definition

any such optically active or racemic form which possesses the property of stimulating GLK directly or inhibiting the GLK/GLKRP interaction. The synthesis of optically active forms may be carried out by standard techniques of organic chemistry well known in the art, for example by synthesis from optically active starting materials or by resolution of a racemic form. It is also to be understood that certain compounds may exist in tautomeric forms and that the invention also relates to any and all tautomeric forms of the compounds of the invention which activate GLK.

Preferred compounds of Formula (I) are those wherein any one or more of the following apply:

(1) The group at the 3 position in Formula (I) is:

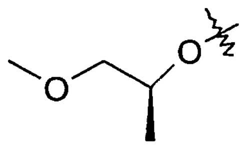

;

(2) $R^2$ is hydrogen;
(3) $R^2$ is fluoro or chloro;
(4) $R^2$ is methyl
(5) $R^2$ is fluoro;
(6) $R^2$ is chloro;
(7) n is 1;
(8) n is 2;
(9) $R^2$ is linked to the phenyl ring to which it is attached at the 3-position relative to the oxygen atom.

According to a further feature of the invention there is provided the following preferred groups of compounds of the invention:

(I) a compound of Formula (Ia)

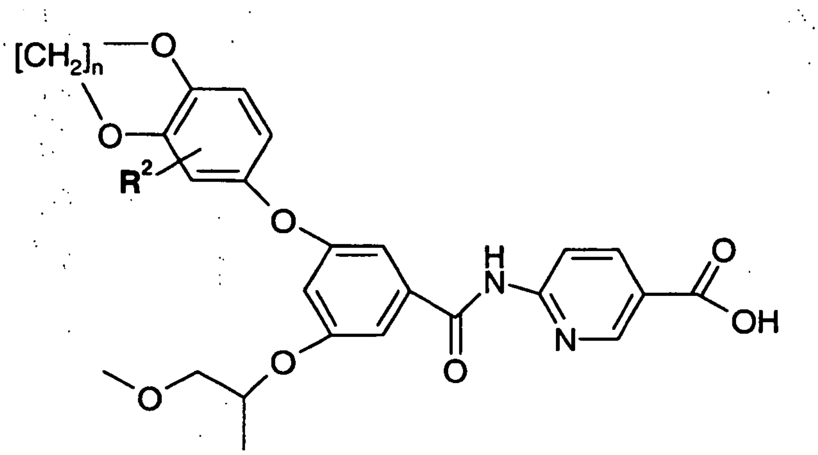

**Formula (Ia)**

wherein:

**n** and $\mathbf{R^2}$ are as defined above in a compound of Formula (I);
or a salt, solvate or in-vivo hydrolysable ester thereof.

(II) a compound of Formula (Ib)

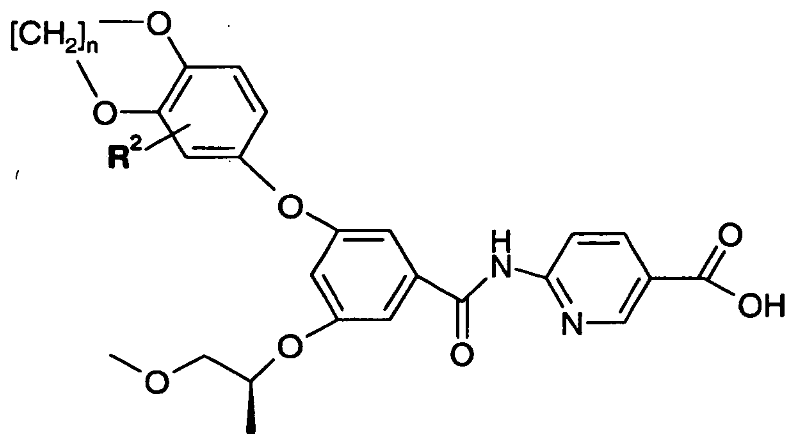

Formula (Ib)

wherein:

**n** and $\mathbf{R^2}$ are as defined above in a compound of Formula (I); or a salt, solvate or in-vivo hydrolysable ester thereof.

(III) a compound of Formula (Ic)

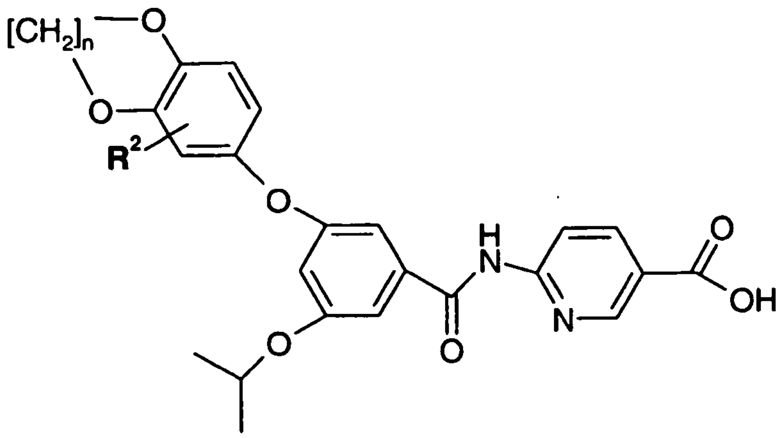

Formula (Ic)

wherein:

**n** and $\mathbf{R^2}$ are as defined above in a compound of Formula (I); or a salt, solvate or in-vivo hydrolysable ester thereof.

(IV) a compound of Formula (Id)

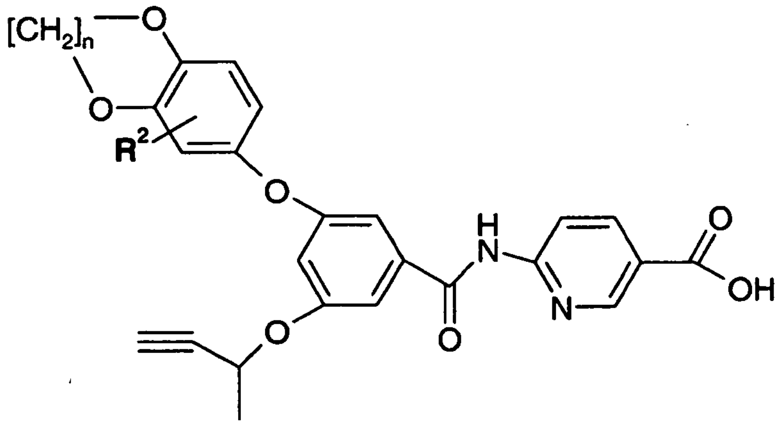

Formula (Id)

wherein:

**n** and $\mathbf{R^2}$ are as defined above in a compound of Formula (I);
or a salt, solvate or in-vivo hydrolysable ester thereof.

(V) a compound of Formula (Ie)

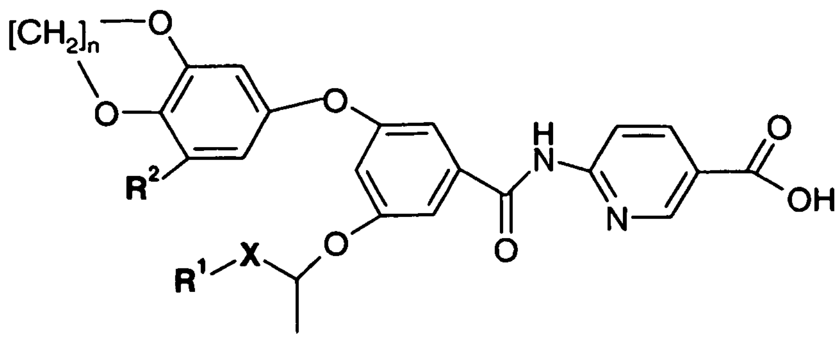

Formula (Ie)

wherein:

**n, X,** $\mathbf{R^1}$ and $\mathbf{R^2}$ are as defined above in a compound of Formula (I);
or a salt, solvate or in-vivo hydrolysable ester thereof.

Preferred compounds of the invention include one or more of the following:

6-{[(3-(2,3-dihydro-1,4-benzodioxin-6-yloxy)-5-{[(1S)-1-methyl-2-(methyloxy)ethyl]oxy}phenyl)carbonyl]amino}pyridine-3-carboxylic acid
6-{[(3-(1,3-benzodioxol-5-yloxy)-5-{[(1S)-1-methyl-2-(methyloxy)ethyl]oxy}phenyl)carbonyl]amino}pyridine-3-carboxylic acid

or a salt, solvate or in-vivo hydrolysable ester thereof.

The compounds of the invention may be administered in the form of a pro-drug. A pro-drug is a bioprecursor or pharmaceutically acceptable compound being degradable in the body to produce a compound of the invention (such as an ester or amide of a compound of the invention, particularly an in vivo hydrolysable ester). Various forms of prodrugs are known in the art. For examples of such prodrug derivatives, see:

a) Design of Prodrugs, edited by H. Bundgaard, (Elsevier, 1985) and Methods in Enzymology, Vol. 42, p. 309-396, edited by K. Widder, et al. (Academic Press, 1985);

b) A Textbook of Drug Design and Development, edited by Krogsgaard-Larsen;

c) H. Bundgaard, Chapter 5 "Design and Application of Prodrugs", by H. Bundgaard p. 113-191 (1991);

d) H. Bundgaard, Advanced Drug Delivery Reviews, 8, 1-38 (1992);

e) H. Bundgaard, et al., Journal of Pharmaceutical Sciences, 77, 285 (1988); and

f) N. Kakeya, et al., Chem Pharm Bull, 32, 692 (1984).

The contents of the above cited documents are incorporated herein by reference.

Examples of pro-drugs are as follows. An in-vivo hydrolysable ester of a compound of the invention containing a carboxy or a hydroxy group is, for example, a pharmaceutically-acceptable ester which is hydrolysed in the human or animal body to produce the parent acid or alcohol. Suitable pharmaceutically-acceptable esters for carboxy include $C_1$ to $C_6$alkoxymethyl esters for example methoxymethyl, $C_1$ to $C_6$alkanoyloxymethyl esters for example pivaloyloxymethyl, phthalidyl esters, $C_3$ to $C_8$cycloalkoxycarbonyloxy$C_1$ to $C_6$alkyl esters for example 1-cyclohexylcarbonyloxyethyl; 1,3-dioxolen-2-onylmethyl esters, for example 5-methyl-1,3-dioxolen-2-onylmethyl; and $C_{1-6}$alkoxycarbonyloxyethyl esters.

An in-vivo hydrolysable ester of a compound of the invention containing a hydroxy group includes inorganic esters such as phosphate esters (including phosphoramidic cyclic esters) and α-acyloxyalkyl ethers and related compounds which as a result of the in-vivo hydrolysis of the ester breakdown to give the parent hydroxy group/s. Examples of α-acyloxyalkyl ethers include acetoxymethoxy and 2,2-dimethylpropionyloxy-methoxy. A selection of in-vivo hydrolysable ester forming groups for hydroxy include alkanoyl, benzoyl, phenylacetyl and substituted benzoyl and phenylacetyl, alkoxycarbonyl (to give alkyl carbonate esters), dialkylcarbamoyl and N-(dialkylaminoethyl)-N-alkylcarbamoyl (to give carbamates), dialkylaminoacetyl and carboxyacetyl.

A suitable pharmaceutically-acceptable salt of a compound of the invention is, for example, an acid-addition salt of a compound of the invention which is sufficiently basic, for example, an acid-addition salt with, for example, an inorganic or organic acid, for example hydrochloric, hydrobromic, sulphuric, phosphoric, trifluoroacetic, citric or maleic acid. In addition a suitable pharmaceutically-acceptable salt of a benzoxazinone derivative of the invention which is sufficiently acidic is an alkali metal salt, for example a sodium or potassium salt, an alkaline earth metal salt, for example a calcium or magnesium salt, an ammonium salt or a salt with an organic base which affords a physiologically-acceptable cation, for example a salt with methylamine, dimethylamine, trimethylamine, piperidine, morpholine or tris-(2-hydroxyethyl)amine.

A further feature of the invention is a pharmaceutical composition comprising a compound of Formula (I), (Ia), (Ib), (Ic), (Id) or (Ie) as defined above, or a salt, solvate or in-vivo hydrolysable ester thereof, together with a pharmaceutically-acceptable diluent or carrier.

According to another aspect of the invention there is provided a compound of Formula (I), (Ia), (Ib), (Ic), (Id) or (Ie) as defined above for use as a medicament.

Further according to the invention there is provided a compound of Formula (I), (Ia), (Ib), (Ic), (Id) or (Ie) for use in the preparation of a medicament for treatment of a disease mediated through GLK, in particular type 2 diabetes.

The compound is suitably formulated as a pharmaceutical composition for use in this way.

Specific diseases which may be treated by a compound or composition of the invention include: blood glucose lowering in Diabetes Mellitus type 2 without a serious risk of hypoglycaemia (and potential to treat type 1), dyslipidemia, obesity, insulin resistance, metabolic syndrome X, impaired glucose tolerance.

As discussed above, thus the GLK/GLKRP system can be described as a potential "Diabesity" target (of benefit in both Diabetes and Obesity). Thus, according to another aspect of the invention there is provided the use of a compound of Formula (I), (Ia), (Ib), (Ic), (Id) or (Ie), or salt, solvate or in-vivo hydrolysable ester thereof, in the preparation of a medicament for use in the combined treatment or prevention of diabetes and obesity.

According to another aspect of the invention there is provided the use of a compound of Formula (I), (Ia), (Ib), (Ic), (Id) or (Ie), or salt, solvate or in-vivo hydrolysable ester thereof, in the preparation of a medicament for use in the treatment or prevention of obesity.

The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular or intramuscular dosing or as a suppository for rectal dosing).

The compositions of the invention may be obtained by conventional procedures using conventional pharmaceutical excipients, well known in the art. Thus, compositions intended for oral use may contain, for example, one or more colouring, sweetening, flavouring and/or preservative agents.

Suitable pharmaceutically acceptable excipients for a tablet formulation include, for example, inert diluents

such as lactose, sodium carbonate, calcium phosphate or calcium carbonate, granulating and disintegrating agents such as corn starch or algenic acid; binding agents such as starch; lubricating agents such as magnesium stearate, stearic acid or talc; preservative agents such as ethyl or propyl p-hydroxybenzoate, and anti-oxidants, such as ascorbic acid. Tablet formulations may be uncoated or coated either to modify their disintegration and the subsequent absorption of the active ingredient within the gastrointestinal tract, or to improve their stability and/or appearance, in either case, using conventional coating agents and procedures well known in the art.

Compositions for oral use may be in the form of hard gelatin capsules in which the active ingredient is mixed with an inert solid diluent, for example, calcium carbonate, calcium phosphate or kaolin, or as soft gelatin capsules in which the active ingredient is mixed with water or an oil such as peanut oil, liquid paraffin, or olive oil.

Aqueous suspensions generally contain the active ingredient in finely powdered form together with one or more suspending agents, such as sodium carboxymethylcellulose, methylcellulose, hydroxypropylmethylcellulose, sodium alginate, polyvinyl-pyrrolidone, gum tragacanth and gum acacia; dispersing or wetting agents such as lecithin or condensation products of an alkylene oxide with fatty acids (for example polyoxethylene stearate), or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives (such as ethyl or propyl p-hydroxybenzoate, anti-oxidants (such as ascorbic acid), colouring agents, flavouring agents, and/or sweetening agents (such as sucrose, saccharine or aspartame).

Oily suspensions may be formulated by suspending the active ingredient in a vegetable oil (such as arachis oil, olive oil, sesame oil or coconut oil) or in a mineral oil (such as liquid paraffin). The oily suspensions may also contain a thickening agent such as beeswax, hard paraffin or cetyl alcohol. Sweetening agents such as those set out above, and flavouring agents may be added to provide a palatable oral preparation. These compositions may be preserved by the addition of an anti-oxidant such as ascorbic acid.

Dispersible powders and granules suitable for preparation of an aqueous suspension by the addition of water generally contain the active ingredient together with a dispersing or wetting agent, suspending agent and one or more preservatives. Suitable dispersing or wetting agents and suspending agents are exemplified by those already mentioned above. Additional excipients such as sweetening, flavouring and colouring agents, may also be present.

The pharmaceutical compositions of the invention may also be in the form of oil-in-water emulsions. The oily phase may be a vegetable oil, such as olive oil or arachis oil, or a mineral oil, such as for example liquid paraffin or a mixture of any of these. Suitable emulsifying agents may be, for example, naturally-occurring gums such as gum acacia or gum tragacanth, naturally-occurring phosphatides such as soya bean, lecithin, an esters or partial esters derived from fatty acids and hexitol anhydrides (for example sorbitan monooleate) and condensation products of the said partial esters with ethylene oxide such as polyoxyethylene sorbitan monooleate. The emulsions may also contain sweetening, flavouring and preservative agents.

Syrups and elixirs may be formulated with sweetening agents such as glycerol, propylene glycol, sorbitol, aspartame or sucrose, and may also contain a demulcent, preservative, flavouring and/or colouring agent.

The pharmaceutical compositions may also be in the form of a sterile injectable aqueous or oily suspension, which may be formulated according to known procedures using one or more of the appropriate dispersing or wetting agents and suspending agents, which have been mentioned above. A sterile injectable preparation may also be a sterile injectable solution or suspension in a non-toxic parenterally-acceptable diluent or solvent, for example a solution in 1,3-butanediol.

Compositions for administration by inhalation may be in the form of a conventional pressurised aerosol arranged to dispense the active ingredient either as an aerosol containing finely divided solid or liquid droplets. Conventional aerosol propellants such as volatile fluorinated hydrocarbons or hydrocarbons may be used and the aerosol device is conveniently arranged to dispense a metered quantity of active ingredient.

For further information on formulation the reader is referred to Chapter 25.2 in Volume 5 of Comprehensive Medicinal Chemistry (Corwin Hansch; Chairman of Editorial Board), Pergamon Press 1990.

The amount of active ingredient that is combined with one or more excipients to produce a single dosage form will necessarily vary depending upon the host treated and the particular route of administration. For example, a formulation intended for oral administration to humans will generally contain, for example, from 0.5 mg to 2 g of active agent compounded with an appropriate and convenient amount of excipients which may vary from about 5 to about 98 percent by weight of the total composition. Dosage unit forms will generally contain about 1 mg to about 500 mg of an active ingredient. For further information on Routes of Administration and Dosage Regimes the reader is referred to Chapter 25.3 in Volume 5 of Comprehensive Medicinal Chemistry (Corwin Hansch; Chairman of Editorial Board), Pergamon Press 1990.

The size of the dose for therapeutic or prophylactic purposes of a compound of the Formula (I), (Ia), (Ib), (Ic), (Id) or (Ie) will naturally vary according to the nature and severity of the conditions, the age and sex of the animal or patient and the route of administration, according to well known principles of medicine.

In using a compound of the Formula (I), (Ia), (Ib), (Ic), (Id) or (Ie) for therapeutic or prophylactic purposes it will generally be administered so that a daily dose in the range, for example, 0.5 mg to 75 mg per kg body weight is received, given if required in divided doses. In general lower doses will be administered when a parenteral route is employed. Thus, for example, for intravenous administration, a dose in the range, for example, 0.5 mg to 30 mg per kg body weight will generally be used. Similarly, for administration by inhalation, a dose in the range, for example, 0.5 mg to 25 mg per kg body weight will be used. Oral administration is however preferred.

The elevation of GLK activity described herein may be applied as a sole therapy or in combination with one or more other substances and/or treatments for the indicated being treated. Such conjoint treatment may be achieved by way of the simultaneous, sequential or separate administration of the individual components of the treatment. Simultaneous treatment may be in a single tablet or in separate tablets. For example in the treatment of diabetes mellitus, chemotherapy may include the following main categories of treatment:

1) Insulin and insulin analogues;
2) Insulin secretagogues including sulphonylureas (for example glibenclamide, glipizide), prandial glucose regulators (for example repaglinide, nateglinide);
3) Agents that improve incretin action (for example dipeptidyl peptidase IV inhibitors, and GLP-1 agonists);
4) Insulin sensitising agents including PPARgamma agonists (for example pioglitazone and rosiglitazone), and agents with combined PPARalpha and gamma activity;
5) Agents that modulate hepatic glucose balance (for example metformin, fructose 1, 6 bisphosphatase inhibitors, glycogen phopsphorylase inhibitors, glycogen synthase kinase inhibitors);
6) Agents designed to reduce the absorption of glucose from the intestine (for example acarbose);
7) Agents that prevent the reabsorption of glucose by the kidney (SGLT inhibitors);
8) Agents designed to treat the complications of prolonged hyperglycaemia (for example aldose reductase inhibitors);
9) Anti-obesity agents (for example sibutramine and orlistat);
10) Anti- dyslipidaemia agents such as, HMG-CoA reductase inhibitors (eg statins); PPAR$\alpha$ agonists (fibrates, eg gemfibrozil); bile acid sequestrants (cholestyramine); cholesterol absorption inhibitors (plant stanols, synthetic inhibitors); bile acid absorption inhibitors (IBATi) and nicotinic acid and analogues (niacin and slow release formulations);
11) Antihypertensive agents such as, $\beta$ blockers (eg atenolol, inderal); ACE inhibitors (eg lisinopril); Calcium antagonists (eg. nifedipine); Angiotensin receptor antagonists (eg candesartan), $\alpha$ antagonists and diuretic agents (eg. furosemide, benzthiazide);
12) Haemostasis modulators such as, antithrombotics, activators of fibrinolysis and antiplatelet agents; thrombin antagonists; factor Xa inhibitors; factor VIIa inhibitors); antiplatelet agents (eg. aspirin, clopidogrel); anticoagulants (heparin and Low molecular weight analogues, hirudin) and warfarin;
13) Agents which antagonise the actions of glucagon; and
14) Anti-inflammatory agents, such as non-steroidal anti-inflammatory drugs (eg. aspirin) and steroidal anti-inflammatory agents (eg. cortisone).

According to another aspect of the present invention there is provided individual compounds produced as end products in the Examples set out below and salts, solvates and pro-drugs thereof.

A compound of the invention, or a salt thereof, may be prepared by any process known to be applicable to the preparation of such compounds or structurally related compounds. Functional groups may be protected and deprotected using conventional methods. For examples of protecting groups such as amino and carboxylic acid protecting groups (as well as means of formation and eventual deprotection), see T.W. Greene and P.G.M. Wuts, "Protective Groups in Organic Synthesis", Second Edition, John Wiley & Sons, New York, 1991.

Processes for the synthesis of compounds of Formula (I), (Ia), (Ib), (Ic), (Id) or (Ie) are provided as a further feature of the invention. Thus, according to a further aspect of the invention there is provided a process for the preparation of a compound of Formula (I), (Ia), (Ib), (Ic), (Id) or (Ie) which comprises:

(a) reaction of an acid of Formula (IIIa) or activated derivative thereof with a compound of Formula (IIIb),

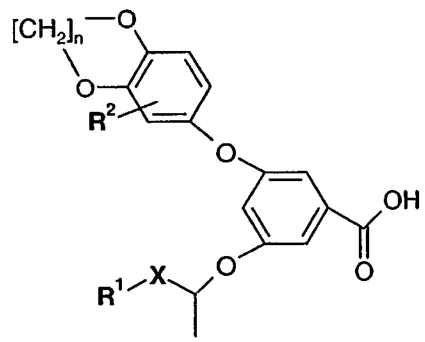
Formula (IIIa)

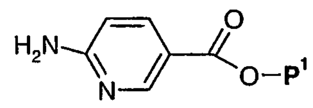
Formula (IIIb);

wherein $P^1$ is hydrogen or a protecting group

or

(b) de-protection of a compound of Formula (IIIc),

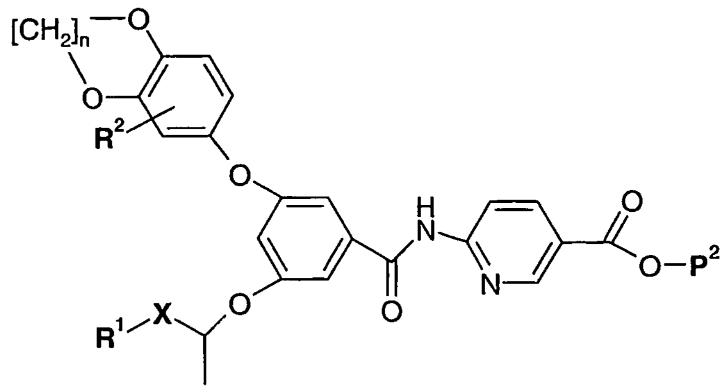
Formula (IIIc)

wherein $P^2$ is a protecting group; or

(c) reaction of a compound of Formula (IIId) with a compound of Formula (IIIe),

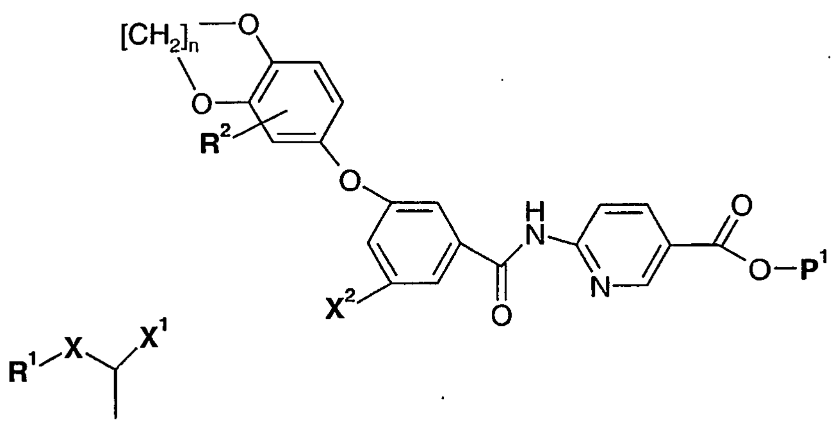
Formula (IIId) Formula (IIIe)

wherein $X^1$ is a leaving group and $X^2$ is a hydroxyl group or $X^1$ is a hydroxyl group and $X^2$ is a leaving group and

wherein **$P^1$** is hydrogen or a protecting group; or

(d) reaction of a compound of Formula (IIIf) with a compound of Formula (IIIg)

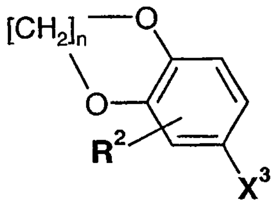

Formula (IIIf)

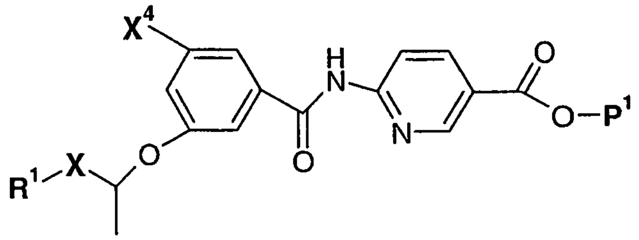

Formula (IIIg)

wherein **$X^3$** is a leaving group or an organometallic reagent and **$X^4$** is a hydroxyl group or **$X^3$** is a hydroxyl group and **$X^4$** is a leaving group or an organometallic reagent wherein **$P^1$** is hydrogen or a protecting group; or

(e) reaction of a compound of Formula (IIIh) with a compound of Formula (IIIi),

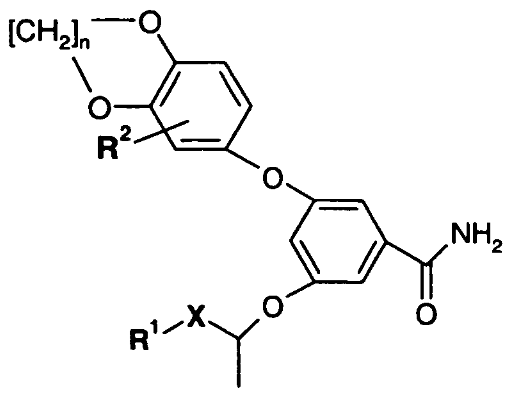

Formula (IIIh)

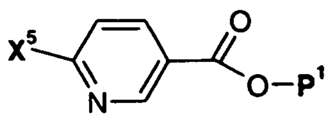

Formula (IIIi);

wherein **$X^5$** is a leaving group and wherein **$P^1$** is hydrogen or a protecting group;

and thereafter, if necessary:

i) converting a compound of Formula (I) into another compound of Formula (I);
ii) removing any protecting groups;
iii) forming a salt, in-vivo hydrolysable ester or solvate thereof.

Suitable leaving groups for processes a) to e) are well known to the skilled person and include for example activated hydroxy leaving groups (such as mesylate and tosylate groups) and halo leaving groups such as fluoro, chloro or bromo.

Compounds of formulae (IIIa) to (IIIi) are commercially available, or may be made by any convenient process known in the art and/or as illustrated in the Examples herein. In general it will be appreciated that any aryl-O or alkyl-O bond may be formed by nucleophilic substitution or metal catalysed processes, optionally in the presence of a suitable base.

Specific reaction conditions for the above reactions are as follows, wherein when $P^1$ is a protecting group $P^1$ is preferably $C_{1-4}$alkyl, for example methyl or ethyl:

*Process a)* - coupling reactions of amino groups with carboxylic acids to form an amide are well known in the art. For example,

(i) using an appropriate coupling reaction, such as a carbodiimide coupling reaction performed with EDAC in the presence of DMAP in a suitable solvent such as DCM, chloroform or DMF at room temperature; or

(ii) reaction in which the carboxylic group is activated to an acid chloride by reaction with oxalyl chloride in the presence of a suitable solvent such as methylene chloride. The acid chloride can then be reacted with a compound of Formula IIIb in the presence of a base, such as triethylamine or pyridine, in a suitable solvent such as chloroform or DCM at a temperature between 0°C and room temperature.

*Process b)* - de-protection reactions are well known in the art. Examples of $P^1$ include $C_{1-6}$alkyl and benzyl. Wherein $P^1$ is an $C_{1-6}$alkyl, the reaction can be performed in the presence of sodium hydroxide in the suitable solvent such as THF/water.

*Process c)* - compounds of Formula (IIId) and (IIIe) can be reacted together in a suitable solvent, such as DMF or THF, with a base such as sodium hydride or potassium tert-butoxide, at a temperature in the range 0 to 100°C, optionally using metal catalysis such as palladium(II)acetate, palladium on carbon, copper(II)acetate or copper(I) iodide; Alternatively, compounds of Formula (IIId) and (IIIe) can be reacted together in a suitable solvent, such as THF or DCM, with a suitable phosphine such as triphenylphosphine, and azodicarboxylate such as diethylazodicarboxylate;

*Process d)* - compounds of Formula (IIId) and (IIIe) can be reacted together in a suitable solvent, such as DMF or THF, with a base such as sodium hydride or potassium tert-butoxide, at a temperature in the range 0 to 100°C, optionally using metal catalysis such as palladium(II)acetate, palladium on carbon, copper(II)acetate or copper(I) iodide;

*Process e)* - reaction of a compound of Formula (IIIh) with a compound of Formula (IIIi) can be performed in a polar solvent, such as DMF or a non-polar solvent such as THF with a strong base, such as sodium hydride or potassium *tert*-butoxide at a temperature between 0 and 100°C, optionally using metal catalysis, such as palladium(II)acetate, palladium on carbon, copper(II)acetate or copper(I)iodide.

During the preparation process, it may be advantageous to use a protecting group for a functional group within the molecule. Protecting groups may be removed by any convenient method as described in the literature or known to the skilled chemist as appropriate for the removal of the protecting group in question, such methods being chosen so as to effect removal of the protecting group with minimum disturbance of groups elsewhere in the molecule.

Specific examples of protecting groups are given below for the sake of convenience, in which "lower" signifies that the group to which it is applied preferably has 1-4 carbon atoms. It will be understood that these examples are not exhaustive. Where specific examples of methods for the removal of protecting groups are given below these are similarly not exhaustive. The use of protecting groups and methods of deprotection not specifically mentioned is of course within the scope of the invention.

A carboxy protecting group may be the residue of an ester-forming aliphatic or araliphatic alcohol or of an ester-forming silanol (the said alcohol or silanol preferably containing 1-20 carbon atoms). Examples of carboxy protecting groups include straight or branched chain (1-12C)alkyl groups (e.g. isopropyl, t-butyl); lower alkoxy lower alkyl groups (e.g. methoxymethyl, ethoxymethyl, isobutoxymethyl; lower aliphatic acyloxy lower alkyl groups, (e.g. acetoxymethyl, propionyloxymethyl, butyryloxymethyl, pivaloyloxymethyl); lower alkoxycarbonyloxy lower alkyl groups (e.g. 1-methoxycarbonyloxyethyl, 1-ethoxycarbonyloxyethyl); aryl lower alkyl groups (e.g. p-methoxybenzyl, o-nitrobenzyl, p-nitrobenzyl, benzhydryl and phthalidyl); tri(lower alkyl)silyl groups (e.g. trimethylsilyl and t-butyldimethylsilyl); tri(lower alkyl)silyl lower alkyl groups (e.g. trimethylsilylethyl); and (2-6C)alkenyl groups (e.g. allyl and vinylethyl).

Methods particularly appropriate for the removal of carboxyl protecting groups include for example acid-, metal- or enzymically-catalysed hydrolysis.

Examples of hydroxy protecting groups include lower alkenyl groups (e.g. allyl); lower alkanoyl groups (e.g. acetyl); lower alkoxycarbonyl groups (e.g. t-butoxycarbonyl); lower alkenyloxycarbonyl groups (e.g. allyloxycarbonyl); aryl lower alkoxycarbonyl groups (e.g. benzoyloxycarbonyl, p-methoxybenzyloxycarbonyl, o-nitrobenzyloxycarbonyl, p-nitrobenzyloxycarbonyl); tri lower alkyl/arylsilyl groups (e.g. trimethylsilyl, t-butyldimethylsilyl, t-butyldiphenylsilyl); aryl lower alkyl groups (e.g. benzyl) groups; and triaryl lower alkyl groups (e.g. triphenylmethyl).

Examples of amino protecting groups include formyl, aralkyl groups (e.g. benzyl and substituted benzyl, e.g. p-methoxybenzyl, nitrobenzyl and 2,4-dimethoxybenzyl, and triphenylmethyl); di-p-anisylmethyl and furylmethyl groups; lower alkoxycarbonyl (e.g. t-butoxycarbonyl); lower alkenyloxycarbonyl (e.g. allyloxycarbonyl); aryl lower alkoxycarbonyl groups (e.g. benzyloxycarbonyl, p-methoxybenzyloxycarbonyl, o-nitrobenzyloxycarbonyl, p-nitrobenzyloxycarbonyl; trialkylsilyl (e.g. trimethylsilyl and t-butyldimethylsilyl); alkylidene (e.g. methylidene); benzylidene and substituted benzylidene groups.

Methods appropriate for removal of hydroxy and amino protecting groups include, for example, acid-, base, metal- or enzymically-catalysed hydrolysis, or photolytically for groups such as o-nitrobenzyloxycarbonyl, or with fluoride ions for silyl groups.

Examples of protecting groups for amide groups include aralkoxymethyl (e.g. benzyloxymethyl and substituted benzyloxymethyl); alkoxymethyl (e.g. methoxymethyl and trimethylsilylethoxymethyl); tri alkyl/arylsilyl (e.g. trimethylsilyl, t-butyldimethylsily, t-butyldiphenylsilyl); tri alkyl/arylsilyloxymethyl (e.g. t-butyldimethylsilyloxymethyl, t-butyldiphenylsilyloxymethyl); 4-alkoxyphenyl (e.g. 4-methoxyphenyl); 2,4-di(alkoxy)phenyl (e.g. 2,4-dimethoxyphenyl); 4-alkoxybenzyl (e.g. 4-methoxybenzyl); 2,4-di(alkoxy)benzyl (e.g. 2,4-di(methoxy)benzyl); and alk-1-enyl (e.g. allyl, but-1-enyl and substituted vinyl e.g. 2-phenylvinyl).

Aralkoxymethyl, groups may be introduced onto the amide group by reacting the latter group with the appropriate aralkoxymethyl chloride, and removed by catalytic hydrogenation. Alkoxymethyl, tri alkyl/arylsilyl and tri alkyl/silyloxymethyl groups may be introduced by reacting the amide with the appropriate chloride and removing with acid; or in the case of the silyl containing groups, fluoride ions. The alkoxyphenyl and alkoxybenzyl groups are conveniently introduced by arylation or alkylation with an appropriate halide and removed by oxidation with ceric ammonium nitrate. Finally alk-1-enyl groups may be introduced by reacting the amide with the appropriate aldehyde and removed with acid.

The following examples are for illustration purposes and are not intended to limit the scope of this application. Each exemplified compound represents a particular and independent aspect of the invention. In the following non-limiting Examples, unless otherwise stated:

(i) evaporations were carried out by rotary evaporation in *vacuo* and work-up procedures were carried out after removal of residual solids such as drying agents by filtration;
(ii) operations were carried out at room temperature, that is in the range 18-25°C and under an atmosphere of an inert gas such as argon or nitrogen;
(iii) yields are given for illustration only and are not necessarily the maximum attainable;
(iv) the structures of the end-products of the Formula (I) were confirmed by nuclear (generally proton) magnetic resonance (NMR) and mass spectral techniques; proton magnetic resonance chemical shift values were measured on the delta scale and peak multiplicities are shown as follows: s, singlet; d, doublet; t, triplet; m, multiplet; br, broad; q, quartet, quin, quintet;
(v) intermediates were not generally fully characterised and purity was assessed by thin layer chromatography (TLC), high-performance liquid chromatography (HPLC), infra-red (IR) or NMR analysis; and
(vi) Biotage cartridges refer to pre-packed silica cartridges (from 40g up to 400g), eluted using a biotage pump and fraction collector system; Biotage UK Ltd, Hertford, Herts, UK.

Abbreviations

| | |
|---|---|
| DCM | dichloromethane; |
| DEAD | diethyldiazocarboxylate; |
| DIAD | di-i-propyl azodicarboxylate; |
| DMAP | 4-(*N.N*-dimethylamino)pyridine |
| DMSO | dimethyl sulphoxide; |
| DMF | dimethylformamide; |
| EDAC | 1-(3-dimethylaminopropyl)-3-ethylcarbodiimide hydrochloride; |
| HPMC | Hydroxypropylmethylcellulose; |
| LCMS | liquid chromatography / mass spectroscopy; |
| RT | room temperature; and |
| THF | tetrahydrofuran. |

**EXAMPLE 1**

**6-{[(3-(2,3-dihydro-1,4-benzodioxin-6-yloxy)-5-{[(1*S*)-1-methyl-2-(methyloxy)ethyl]oxy}phenyl)carbonyl]amino}pyridine-3-carboxylic acid**

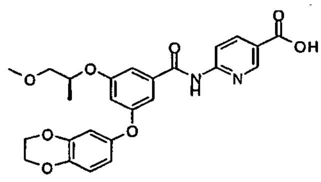

To a solution of methyl-6-{[(3-(2,3-dihydro-1,4-benzodioxin-6-yloxy)-5-{[(1*S*)-1-methyl-2-(methyloxy)ethyl]oxy}phenyl)carbonyl]amino}pyridine-3-carboxylate (0.05 mmol) in THF (4 mL) was added lithium hydroxide monohydrate (2.5 equivalents) in water (2 mL). The mixture was stirred at ambient temperature for 4 hours. The pH of the reaction was adjusted to <7.0 and the THF removed *in vacuo* and replaced with water (8 mL). The resulting solid was filtered, washed with water and dried to give 6-{[(3-(2,3-dihydro-1,4-benzodioxin-6-yloxy)-5-{[(1*S*)-1-methyl-2-(methyloxy)ethyl]oxy}phenyl)carbonyl]amino} pyridine-3-carboxylic acid.
m/z 481 $(M+H)^+$.

Methyl-6-{[(3-(2,3-dihydro-1,4-benzodioxin-6-yloxy)-5-{[(1*S*)-1-methyl-2-(methyloxy)ethyl]oxy}phenyl)carbonyl]amion}pyridine-3-carboxylate

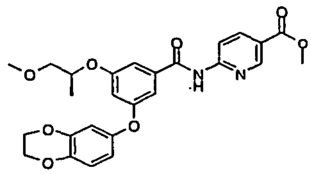

A solution of Methyl-6-{[3-hydroxy-5-{[(1*S*)-1-methyl-2-(methyloxy)ethyl]oxy} phenyl)carbonyl]amino}pyridine-3-carboxylate (0.75 mmol), 1,4-benzodioxin-6-boronic acid (0.75 mmol), copper (II) acetate (0.75mmol), triethylamine (3.75 mmol) and freshly activated 4A molecular sieves (1 g) in DCM (10 mL) was stirred at ambient temperature and under ambient atmosphere for 2 days. The reaction mixture was filtered, the DCM removed *in vacuo* and the residual oil partitioned between ethyl acetate and hydrochloric acid (1N). The ethyl acetate layer was separated, washed with aqueous sodium hydrogen carbonate solution, brine, dried ($MgSO_4$) and evaporated to a residue which was chromatographed on silica with 40% ethyl acetate in iso-hexane as eluant to give Methyl-6-{[(3-(2,3-dihydro-1,4-benzodioxin-6-yloxy)-5-{[(1*S*)-1-methyl-2-(methyloxy)ethyl]oxylphenyl)carbonyl]amino} pyridine-3-carboxylate.
m/z 495 $(M+H)^+$. $^1H$ NMR ($CDCl_3$): 1.3 (d, 3H), 3.4 (s, 3H), 3.5 (m, 2H), 4.0 (s, 3H), 4.3 (s, 4H), 4.6 (m, 1H), 6.6 (m, 2H), 6.75 (m, 1H), 6.85 (dd, 1H), 7.0 (m, 1H), 7.2 (m, 1H), 8.3 (m, 2H), 8.7 (s, 1H), 8.95 (s, 1H).

## EXAMPLE 2

**6-{[(3-(1,3-benzodioxol-5-yloxy)-5-{[(1*S*)-1-methyl-2-(methyloxy)ethyl]oxy}phenyl)carbonyl]amino}pyridine-3-carboxylic acid**

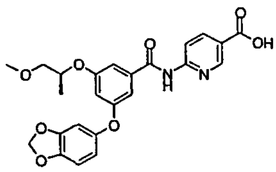

To a 0.1M solution of methyl-6-{[(3-(1,3-benzodioxol-5-yloxy)-5-{[(1*S*)-1-methyl-2-(methyloxy)ethyl]oxy}phenyl)carbonyl]amino}pyridine-3-carboxylate was added a 0.5N solution of sodium hydroxide (5 equivalents). The mixture was stirred at room temperature for 4 hours. The organics were removed *in vacuo* and the residue diluted with water and acidified with hydrochloric acid (2N). The resultant precipitate was filtered, washed with water and dried *in vacuo* to give 6-{[(3-(1,3-benzodioxol-5-yloxy)-5-{[(1S)-1-methyl-2-(methyloxy)ethyl]oxy}phenyl)carbonyl]amino}pyridine-3-carboxylic acid.
m/z 467 $(M+H)^+$, 465 $(M-H)^-$; $^1H$ NMR δ ($d_6$-DMSO): 1.22 (d, 3H), 3.28 (s, 3H obscured by solvent peak), 3.46 (m, 2H), 4.74 (s, 1H), 6.04 (s, 2H), 6.56 (d, 1H), 6.73 (d, 1H), 6.92 (d, 1H), 7.09 (s, 1H), 7.37 (s, 1H), 8.26 (s, 2H), 8.86 (s, 1H), 11.15 (s, 1H), 13.12 (brs, 1H).

Methyl-6-{[(3-(1,3-benzodioxol-5-yloxy)-5-{[(1*S*)-1-methyl-2-(methyloxy)ethyl]oxy}phenyl)carbonyl]amino}pyridine-3-carboxylate

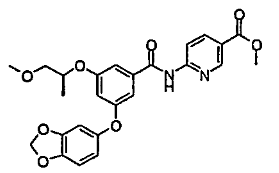

A solution of methyl-6-{[3-hydroxy-5-{[(1*S*)-1-methyl-2-(methyloxy)ethyl]oxy} phenyl)carbonyl]amino}pyridine-3-carboxylate (0.5 mmol), 1,3-benzodioxol-5-ylboronic acid (1.0 mmol), copper (II) acetate (0.5 mmol), triethylamine (2.5 mmol) and freshly activated 4A molecular sieves (500 mg) in DCM (5 mL) was stirred at ambient temperature and under ambient atmosphere for 2 days, additional solvent, molecular sieves and boronic acid (1 equiv) were added and the reaction stirred for a further 4 days. The reaction mixture was concentrated *in vacuo,* the residue triturated with ethyl acetate, filtered and concentrated *in vacuo.* The residue was chromatographed on silica with 20% ethyl acetate in isohexane as eluant to give methyl-6-{[(3-(1,3-benzodioxol-5-yloxy)-5-{[(1S)-1-methyl-2-(methyloxy)ethyl]oxy}phenyl)carbonyl]amino }pyridine-3-carboxylate.
m/z 481 $(M+H)^+$.

**Boronic Acid Synthesis**

The boronic acids used for examples 1 & 2 were either commercially available, or synthesised from commercially available materials as follows. To a solution of the appropriate bromide (10 mmol) in ether (25 mL) at -78°C was added a 1.6M solution of *n*-butyl lithium in hexane (11 mmol). The reaction mixture was stirred at -78°C for 10 minutes, tri-isopropyl borate (11mmol) added and the reaction mixture stirred at -78°C for 30 minutes. The reaction mixture was allowed to come to ambient temperature, stirred for a further 30 minutes then quenched with water (20 mL). The aqueous layer was separated, washed with ether (25 mL) and acidified to pH 1 with concentrated hydrochloric acid. The resulting solid was filtered off, washed with water and dried to give the desired boronic acid.

Methyl 6-{[(3-hydroxy-5-{[(*1S*)-1-methyl-2-(methyloxy)ethyl]oxy}phenyl)carbonyl}amino}pyridine-3-carboxylate

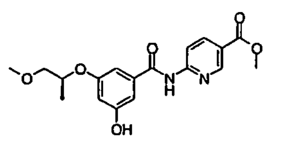

To a stirred solution of methyl 6-[({3-{ [(*1S*)-1-methyl-2-(methyloxy)ethyl]oxy}-5-[(phenylmethyl)oxy]phenyl}carbonyl)amino]pyridine-3-carboxylate (0.038 mol) in THF (85 mL) was added methanol (85 mL). Palladium on charcoal catalyst (1.7g of 10% w/w) was added under an argon atmosphere, and the resulting suspension stirred at ambient temperature overnight in an atmosphere of hydrogen. The catalyst was filtered off through celite, washed with THF, and the filtrate evaporated to give a pale brown solid. This was triturated with ether to give the desired compound (72% yield).
m/z 361 $(M+H)^+$, 359 $(M-H)^-$; $^1H$ NMR δ ($d_6$-DMSO): 1.25 (d, 3H), 3.3 (s, 3H), 3.45 (m, 2H), 3.85 (s, 3H), 4.65 (m, 1H), 6.55 (m, 1H), 6.95 (m, 1H), 7.1 (m, 1H), 8.3 (m, 2H), 8.9 (m, 1H), 11.0, (s, 1H).

Methyl 6-[({3-{[(*1S*)-1-methyl-2-(methyloxy)ethyl]oxy}-5-[(phenylmethyl)oxy]phenyl}carbonyl)amino]pyridine-3-carboxylate

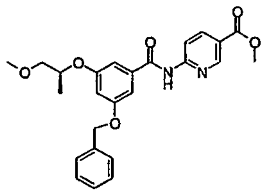

To a stirred solution of 3-{[(*1S*)-1-methyl-2-(methyloxy)ethyl]oxy}-5-[(phenylmethyl)oxy]benzoic acid (75.9 mmol) in DCM (250 mL) containing DMF (1 mL), oxalyl chloride was added dropwise under argon (151.7 mmol), and the resulting solution stirred for 4 hours. The solution was then evaporated *in vacuo,* azeotroped with more DCM (3x100 mL), and the residue dried under high vacuum to give the acid chloride, which was used without characterisation.

The acid chloride from above (approx. 75.9 mmol) was dissolved in THF (100 mL) and added under argon to a stirred solution of methyl 6-aminonicotinate (91.1 mmol) in a mixture of THF (100 mL) and pyridine (100 mL). The reaction mixture was stirred overnight, and then most of the solvent removed *in vacuo.* The residue was taken up in ethyl acetate (250 mL), and the suspension washed sequentially with 1M citric acid (2 portions, until washings acidic) and brine; the resulting solution was dried ($MgSO_4$) and evaporated to give the crude product as a brown gum. This was chromatographed (400 g Biotage silica cartridge, eluting with hexane containing ethyl acetate, 20% v/v) to give the desired compound (50% yield).
m/z 451.47 $(M+H)^+$, 449.48 $(M-H)^-$ ; $^1$H NMR δ ($d_6$-DMSO): 1.21 (d, 3H), 3.47 (m, 2H), 3.86 (s, 3H), 3.72 (m, 1H), 5.16 (s, 2H), 6.78 (t, 1H), 7.23 (s, 1H), 7.29 (s, 1H), 7.31-7.49 (m, 5H), 8.32 (s, 2H), 8.90 (app t, 1H), 11.15 (s, 1H).

3-{[(*1S*)-1-Methyl-2-(methyloxy)ethylloxyl-5-[(phenylmethyl)oxy]benzoic acid

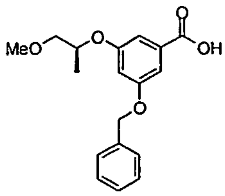

A solution of methyl 3-{[(*1S*)-1-methyl-2-(methyloxy)ethyl]oxy}-5-[(phenylmethyl)oxy]benzoate (77.4 mmol) in a mixture of THF (232 mL) and methanol (232 mL) was treated with a solution of sodium hydroxide (2N) (232 mmol), and the reaction mixture stirred for 4 hours at ambient temperature. The resulting solution was diluted with water (250 mL) and most of the organic solvent removed *in vacuo.* The resulting suspension was washed with diethyl ether (3x200 mL) and the washings discarded. The resulting aqueous solution was acidified to pH 4 with hydrochloric acid solution (2M) and extracted with ethyl acetate (2x200 mL); the extracts were combined, washed with brine, dried ($MgSO_4$) and evaporated to give the desired compound (99% yield).
$^1$H NMR δ ($d_6$-DMSO): 1.20 (d, 3H), 3.46 (m, 2H), 4.64 (m, 1H), 5.15 (s, 2H), 6.83 (app t, 1H), 7.06 (s, 1H), 7.13 (s, 1H), 7.30-7.49 (m, 5H), 12.67 (brs, 1H).

Methyl 3-{[(*1S*)-1-methyl-2-(methyloxy)ethyl]oxy}-5-[(phenylmethyl)oxy]benzoate

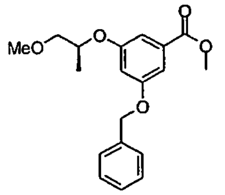

To a solution of methyl 3-hydroxy-5-[(phenylmethyl)oxy]benzoate (77.4 mmol) in THF was added polymer-supported triphenylphosphine (51.7g of 3 mmol/g loading, 155mmol) and (R)-(-)-1-methoxy-2-propanol (102 mmol). The stirred solution was blanketed with argon and cooled in an ice bath; a solution of diisopropyl azodicarboxylate (116 mmol) was added dropwise from a syringe over 10 minutes. After addition the solution was stirred for 20 minutes and then filtered, washing the residue with THF (500 mL); the filtrate and washings were combined and evaporated to give crude desired compound which was used in the next step without further purification.
$^1$H NMR δ ($d_6$-DMSO): 3.26 (s, 3H), 3.44 (m, 2H), 3.82 (s, 3H), 4.63 (m, 1H), 5.14 (s, 2H), 6.85 (s, 1H), 7.05 (s, 1H), 7.11 (s, 1H), 7.30-7.47 (m, 5H); the spectrum also contained signals consistent with a small amount of bis(1-methylethyl) hydrazine-1,2-dicarboxylate.

Methyl 3-hydroxy-5-[(phenylmethyl)oxy]benzoate

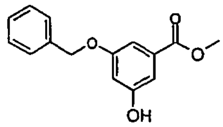

To a stirred solution of methyl 3,5-dihydroxybenzoate (5.95 mol) in DMF (6 L) was added potassium carbonate (9 mol), and the suspension stirred at ambient temperature under argon. To this was added benzyl bromide (8.42 mol) slowly over 1 hour, with a slight exotherm, and the reaction mixture stirred overnight at ambient temperature. It was then quenched cautiously with ammonium chloride solution (5 L) followed by water (35 L). The aqueous suspension was extracted with DCM (1x3 L and 2x5 L). The combined extracts were washed with water (10 L) and dried overnight ($MgSO_4$). The solution was evaporated *in vacuo,* and the crude product chromatographed in three batches (flash column, 3x2 kg silica, eluting with a gradient consisting of hexane containing 10% DCM, to neat DCM, to DCM containing 50% ethyl acetate) to eliminate starting material; the crude eluant was then chromatographed in 175 g batches (Amicon HPLC, 5 kg normal-phase silica, eluting with *iso*-hexane containing 20% v/v of ethyl acetate) to give the desired compound (21% yield).
$^1$H NMR δ ($d_6$-DMSO): 3.8 (s, 3H), 5.1 (s, 2H), 6.65 (m, 1H), 7.0 (m, 1H), 7.05 (m, 1H), 7.3-7.5 (m, 5H), 9.85 (brs, 1H).

**BIOLOGICAL**

Tests:

The biological effects of the compounds of formula (Ia), (Ib), (Ic), (Id) or (Ie) may be tested in the following way:

(1) Enzymatic activity of GLK may be measured by incubating GLK, ATP and glucose. The rate of product formation may be determined by coupling the assay to a G-6-P dehydrogenase, NADP/NADPH system and measuring the linear increase in optical density at 340nm (Matschinsky et al 1993). Activation of GLK by compounds can be assessed using this assay in the presence or absence of GLKRP (GLK regulatory protein) as described in Brocldehurst et al (Diabetes 2004, 53, 535-541).

(2) A GLK/GLKRP binding assay for measuring the binding interactions between GLK and GLKRP. The method may be used to identify compounds which modulate GLK by modulating the interaction between GLK and GLKRP. GLKRP and GLK are incubated with an inhibitory concentration of F-6-P, optionally in the presence of test compound, and the extent of interaction between GLK and GLKRP is measured. Compounds which either displace F-6-P or in some other way reduce the GLK/GLKRP interaction will be detected by a decrease in the amount of GLK/GLKRP complex formed. Compounds which promote F-6-P binding or in some other way enhance the GLK/GLKRP interaction will be detected by an increase in the amount of GLK/GLKRP complex formed. A specific example of such a binding assay is described below

*GLK/GLKRP scintillation proximity assay*

Recombinant human GLK and GLKRP were used to develop a "mix and measure" 96 well SPA (scintillation proximity assay) as described in WO01/20327 (the contents of which are incorporated herein by reference). GLK (Biotinylated) and GLKRP are incubated with streptavidin linked SPA beads (Amersham) in the presence of an inhibitory concentration of radiolabelled [3H]F-6-P (Amersham Custom Synthesis TRQ8689), giving a signal. Compounds which either displace the F-6-P or in some other way disrupt the GLK / GLKRP binding interaction will cause this signal to be lost.
Binding assays were performed at room temperature for 2 hours. The reaction mixtures contained 50mM Tris-HCl (pH 7.5), 2mM ATP, 5mM $MgCl_2$, 0.5mM DTT, recombinant biotinylated GLK (0.1 mg), recombinant GLKRP (0.1 mg), 0.05mCi [3H] F-6-P (Amersham) to give a final volume of 100ml. Following incubation, the extent of GLK/GLKRP complex formation was determined by addition of 0.1mg/well avidin linked SPA beads (Amersham) and scintillation counting on a Packard TopCount NXT.

(3) A F-6-P / GLKRP binding assay for measuring the binding interaction between GLKRP and F-6-P. This method may be used to provide further information on the mechanism of action of the compounds. Compounds identified in the GLK/GLKRP binding assay may modulate the interaction of GLK and GLKRP either by displacing F-6-P or by modifying the GLK/GLKRP interaction in some other way. For example, protein-protein interactions are generally known to occur by interactions through multiple binding sites. It is thus possible that a compound which modifies the interaction between GLK and GLKRP could act by binding to one or more of several different binding sites.

The F-6-P / GLKRP binding assay identifies only those compounds which modulate the interaction of GLK and GLKRP by displacing F-6-P from its binding site on GLKRP.
GLKRP is incubated with test compound and an inhibitory concentration of F-6-P, in the absence of GLK, and the extent of interaction between F-6-P and GLKRP is measured. Compounds which displace the binding of F-6-P to GLKRP may be detected by a change in the amount of GLKRP/F-6-P complex formed. A specific example of such a binding assay is described below

*F-6-P / GLKRP scintillation proximity assay*

Recombinant human GLKRP was used to develop a "mix and measure" 96 well scintillation proximity assay) as described in WO01/20327 (the contents of which are incorporated herein by reference). FLAG-tagged GLKRP is incubated with protein A coated SPA beads (Amersham) and an anti-FLAG antibody in the presence of an inhibitory concentration of radiolabelled [3H]F-6-P. A signal is generated. Compounds which displace the F-6-P will cause this signal to be lost. A combination of this assay and the GLK/GLKRP binding assay will allow the observer to identify compounds which disrupt the GLK/GLKRP binding interaction by displacing F-6-P.
Binding assays were performed at room temperature for 2 hours. The reaction mixtures contained 50mM Tris-HCl (pH 7.5), 2mM ATP, 5mM $MgCl_2$, 0.5mM DTT, recombinant FLAG tagged GLKRP (0.1 mg), Anti-Flag M2 Antibody (0.2mg) (IBI Kodak), 0.05mCi [3H] F-6-P (Amersham) to give a final volume of 100ml. Following incubation, the extent of F-6-P/GLKRP complex formation was determined by addition of 0.1mg/well protein A linked SPA beads (Amersham) and scintillation counting on a Packard TopCount NXT.

Production of recombinant GLK and GLKRP:

*Preparation of mRNA*

Human liver total mRNA was prepared by polytron homogenisation in 4M guanidine isothiocyanate, 2.5mM citrate, 0.5% Sarkosyl, 100mM b-mercaptoethanol, followed by centrifugation through 5.7M CsCl, 25mM sodium acetate at 135,000g (max) as described in Sambrook J, Fritsch EF & Maniatis T, 1989.
Poly $A^+$ mRNA was prepared directly using a FastTrack™ mRNA isolation kit (Invitrogen).

*PCR amplification of GLK and GLKRP cDNA sequences*

Human GLK and GLKRP cDNA was obtained by PCR from human hepatic mRNA using established techniques described in Sambrook, Fritsch & Maniatis, 1989. PCR primers were designed according to the GLK and GLKRP cDNA sequences shown in Tanizawa et al 1991 and Bonthron, D.T. *et al* 1994 (later corrected in Warner, J.P. 1995).

*Cloning in Bluescript II vectors*

GLK and GLKRP cDNA was cloned in E. coli using pBluescript II, (Short et al 1998) a recombinant cloning vector system similar to that employed by Yanisch-Perron C *et al* (1985), comprising a colEI-based replicon bearing a polylinker DNA fragment containing multiple unique restriction sites, flanked by bacteriophage T3 and T7 promoter sequences; a filamentous phage origin of replication and an ampicillin drug resistance marker gene.

*Transformations*

E. Coli transformations were generally carried out by electroporation. 400 ml cultures of strains DH5a or BL21 (DE3) were grown in L-broth to an OD 600 of 0.5 and harvested by centrifugation at 2,000g. The cells were washed twice in ice-cold deionised water, resuspended in 1ml 10% glycerol and stored in aliquots at -70°C. Ligation mixes were desalted using Millipore V series™ membranes (0.0025mm) pore size). 40ml of cells were incubated with 1ml of ligation mix or plasmid DNA on ice for 10 minutes in 0.2cm electroporation cuvettes, and then pulsed using a Gene Pulser™ apparatus (BioRad) at 0.5kVcm$^{-1}$, 250mF. Transformants were selected on L-agar supplemented with tetracyline at 10mg/ml or ampicillin at 100mg/ml.

*Expression*

GLK was expressed from the vector pTB375NBSE in E.coli BL21 cells,, producing a recombinant protein containing a 6-His tag immediately adjacent to the N-terminal methionine. Alternatively, another suitable vector is pET21 (+)DNA, Novagen, Cat number 697703. The 6-His tag was used to allow purification of the recombinant protein on a column packed with nickel-nitrilotriacetic acid agarose purchased from Qiagen (cat no 30250).

GLKRP was expressed from the vector pFLAG CTC (IBI Kodak) in E.coli BL21 cells, producing a recombinant protein containing a C-terminal FLAG tag. The protein was purified initially by DEAE Sepharose ion exchange followed by utilisation of the FLAG tag for final purification on an M2 anti-FLAG immunoaffinity column purchased from Sigma-Aldrich (cat no. A1205).

<u>Biotinylation of GLK:</u>

GLK was biotinylated by reaction with biotinamidocaproate N-hydroxysuccinimide ester (biotin-NHS) purchased from Sigma-Aldrich (cat no. B2643). Briefly, free amino groups of the target protein (GLK) are reacted with biotin-NHS at a defined molar ratio forming stable amide bonds resulting in a product containing covalently bound biotin. Excess, nonconjugated biotin-NHS is removed from the product by dialysis. Specifically, 7.5mg of GLK was added to 0.31mg of biotin-NHS in 4mL of 25mM HEPES pH7.3, 0.15M KCl, 1mM dithiothreitol, 1mM EDTA, 1mM $MgCl_2$ (buffer A). This reaction mixture was dialysed against 100mL of buffer A containing a further 22mg of biotin-NHS. After 4hours excess biotin-NHS was removed by extensive dialysis against buffer A.

<u>Measurement of plasma levels and plasma protein binding following oral administration to rats</u>

*Administration of compounds to rats and sampling of plasma*

Planetary Milled compounds [15mins, 500rpm, 5 Zirconium Balls, in a Puluerisette 7 Mill (Glen Creston Ltd, Stanmore, Middlesex, UK)] were suspended in 0.5% HPMC Tween and dosed to High Fat Fed (Research Diets, D12451, ad lib feeding 14 days) Female Alderley Park Zucker or Alderley Park Wistar rats at rate of 5mls/kg, at doses between 0.3 and 10mg/kg by oral gavage.

Samples of plasma were obtained either by conscious blood sampling or terminal blood sampling as follows:

Conscious blood sampling (for compound level or blood chemistry) - Intravenous blood samples were taken from tail vein using 600μl Starstedt Multivette (EDTA) and 22G needle at the required time point. Samples were kept on ice and centrifuged at 3000rpm for 10 minutes within 15-30 minutes of withdrawal. The plasma was aspirated and stored at -20°C

Terminal blood sampling for compound level or blood chemistry - At the end of experiment animals were euthanased by exposure to $CO_2/O_2$. Blood sample were taken by cardiac puncture. Samples were kept on ice and centrifuged at 3000rpm for 10 minutes within 15-30 minutes of withdrawal. The plasma was aspirated and stored at -20°C

*Measurement of compound levels in rat plasma*

25μl of rat plasma was added to wells in a 96 well protein precipitation plate (Varian inc. Palo Alto, California, USA). To each well was added 500μl of acetonitrile, containing 1ug/ml of (3-isopropoxy-5-benzyoxy-benzoyl)amino pyridine 3-carboxylic acid to act as an internal standard, to precipitate the plasma proteins. Then the plasma/solvent mixture was pulled through the precipitation plate under vacuum and the eluent was collected. The eluent was evaporated to dryness using a centrifugal evaporator and reconstituted in 200μl of methanol:water:formic acid (60:40:0.1).

The reconstituted samples were then analysed using high performance liquid chromatography with tandem mass spectrometry detection (HPLC-MS-MS)". HPLC was performed using a Phenomenex Prodigy C8, 50x4.6, 5μm.column (Phenomenex, Macclesfield, UK) at a flow rate of 1ml/minute using an injection volume of 10μl using the following gradient elution profile:

| | |
|---|---|
| Mobile phase A | 0.1% formic acid in water |
| Mobile phase B | 0.1% formic acid in methanol |
| Mobile phase gradient | 0 min 50% A |
| | 0.5 min 5% A |
| | 2.5 min 5% A |
| | 2.6 min 50% A |
| | 3.0 min 50% A. |

Mass spectroscopy was performed using an Applied Biosystems API3000 Mass spectrometer (Applied Biosystems, Foster City, California, USA ). Prior to the running of samples the mass spectrometer was optimised for the structure of the test compound.

The concentration of test samples was determined from the ratio of the peak height of the test sample to the peak height of the internal standard. The concentration of the test sample was calculated with reference to a standard curve relating the ratio to the concentration prepared by using known concentrations of test sample added to samples of rat plasma using (3-isopropoxy-5-benzyoxy-benzoyl)amino pyridine 3-carboxylic acid as an internal standard, treated as described above.

*Measurements of plasma protein binding of compounds*

The plasma protein binding of compounds was measured using the equilibrium dialysis technique (W. Lindner et al, J.Chromatography, 1996, 677, 1-28). Compound was dialysed at a concentration of 20 μM for 18 hours at 37°C with plasma and isotonic phosphate buffer pH 7.4 (1ml of each in the dialysis cell). A Spectrum® 20-cell equilibrium dialyser was used together with Teflon, semi-micro dialysis cells and Spectra/Por®2 membrane discs with a molecular weight cut off 12-14000 Dalton, 47mm (supplied by PerBio Science UK Ltd, Tattenhall, Cheshire). Plasma and buffer samples are removed following dialysis and analysed using HPLCUV/MS (high performance liquid chromatography with UV and mass spec detection) to give the % free level in plasma.

*Estimation of plasma half-life*

The plasma half-life is the time taken for the concentration of compound in the plasma to decline to half of its original value. This is typically determined following intravenous administration of the test compound, followed by measurement of the compound concentrations in plasma samples as described above. The plasma half-life is estimated from a semilogarithmic plot, plotting the log of the plasma concentration (ln*Cp*) against sample time (t, linear). The apparent first-order elimination rate constant, k, is equal to the slope of the line, and the elimination half-life (*t*½) is the reciprocal of the rate constant (Gibaldi, M and Perrier, D, 1975 Pharmacokinetics, Marcel Dekker, New York):

$$t_{1/2} = \frac{1}{k}$$

Compounds of the invention have the following characteristics:

(i) an activating activity for glucokinase with an $EC_{50}$ of less than about 200nM;
(ii) a percentage free in plasma of between about 0.04% and about 1%;
(iii) a peak blood levels (including both bound and free) of between about 0.3μM and about 10μM for a normalised does of 1mg of compound per kilogram of rat body weight; and
(iv) a half life in plasma (t½) of at least about 1 hour.

For example, Example 2 has the following values:

| $EC_{50}$ | % free in plasma | Peak Blood levels | t½ |
|---|---|---|---|
| 60nM | 0.26% | 2.7μM | 6.4 hours |

**REFERENCES**

1 Printz, R. L., Magnuson, M. A. and Granner, D. K. (1993) Annual Review of Nutrition 13, 463-96
2 DeFronzo, R. A. (1988) Diabetes 37, 667-87
3 Froguel, P., Zouali, H., Vionnet, N., Velho, G., Vaxillaire, M., Sun, F., Lesage, S., Stoffel, M., Takeda, J. and Passa, P. (1993) New England Journal of Medicine 328, 697-702
4 Bell, G. I., Pilkis, S. J., Weber, I. T. and Polonsky, K. S. (1996) Annual Review of Physiology 58, 171-86
5 Velho, G., Petersen, K. F., Perseghin, G., Hwang, J. H., Rothman, D. L., Pueyo, M. E., Cline, G. W., Froguel, P. and Shulman, G. I. (1996) Journal of Clinical Investigation 98, 1755-61
6a Gloyn, A.L., Noordam, K., Willemsen, M.A.A.P., Ellard, S., Lam, W.W.K., Campbell, I. W., Midgley, P., Shiota, C., Buettger, C., Magnuson, M.A., Matschinsky, F.M., and Hattersley, A.T.; Diabetes 52: 2433-2440
6 Christesen, H. B., Jacobsen, B. B., Odili, S., Buettger, C., Cuesta-Munoz, A., Hansen, T., Brusgaard, K., Massa, O., Magnuson, M. A., Shiota, C., Matschinsky, F. M. and Barbetti, F. (2002) Diabetes 51, 1240-6
7 Glaser, B., Kesavan, P., Heyman, M., Davis, E., Cuesta, A., Buchs, A., Stanley, C. A., Thornton, P. S., Permutt, M. A., Matschinsky, F. M. and Herold, K. C. (1998) New England Journal of Medicine 338, 226-30
8 Caro, J. F., Triester, S., Patel, V. K., Tapscott, E. B., Frazier, N. L. and Dohm, G. L. (1995) Hormone & Metabolic Research 27, 19-22
9 Desai, U. J., Slosberg, E. D., Boettcher, B. R., Caplan, S. L., Fanelli, B., Stephan, Z., Gunther, V. J., Kaleko, M. and Connelly, S. (2001) Diabetes 50, 2287-95
10 Shiota, M., Postic, C., Fujimoto, Y., Jetton, T. L., Dixon, K., Pan, D., Grimsby, J., Grippo, J. F., Magnuson, M. A. and Cherrington, A. D. (2001) Diabetes 50, 622-9
11 Ferre, T., Pujol, A., Riu, E., Bosch, F. and Valera, A. (1996) Proceedings of the National Academy of Sciences of the United States of America 93, 7225-30
12 Seoane, J., Barbera, A., Telemaque-Potts, S., Newgard, C. B. and Guinovart, J. J. (1999) Journal of Biological Chemistry 274, 31833-8
13 Moore, M. C., Davis, S. N., Mann, S. L. and Cherrington, A. D. (2001) Diabetes Care 24, 1882-7
14 Alvarez, E., Roncero, I., Chowen, J. A., Vazquez, P. and Blazquez, E. (2002) Journal of Neurochemistry 80, 45-53
15 Lynch, R. M., Tompkins, L. S., Brooks, H. L., Dunn-Meynell, A. A. and Levin, B. E. (2000) Diabetes 49, 693-700
16 Roncero, I., Alvarez, E., Vazquez, P. and Blazquez, E. (2000) Journal of Neurochemistry 74, 1848-57
17 Yang, X. J., Kow, L. M., Funabashi, T. and Mobbs, C. V. (1999) Diabetes 48, 1763-1772
18 Schuit, F. C., Huypens, P., Heimberg, H. and Pipeleers, D. G. (2001) Diabetes 50, 1-11
19 Levin, B. E. (2001) International Journal of Obesity 25, supplement 5, S68-S72.
20 Alvarez, E., Roncero, I., Chowen, J. A., Thorens, B. and Blazquez, E. (1996) Journal of Neurochemistry 66, 920-7
21 Mobbs, C. V., Kow, L. M. and Yang, X. J. (2001) American Journal of Physiology - Endocrinology & Metabolism 281, E649-54
22 Levin, B. E., Dunn-Meynell, A. A. and Routh, V. H. (1999) American Journal of Physiology 276, R1223-31
23 Spanswick, D., Smith, M. A., Groppi, V. E., Logan, S. D. and Ashford, M. L. (1997) Nature 390, 521-5
24 Spanswick, D., Smith, M. A., Mirshamsi, S., Routh, V. H. and Ashford, M. L. (2000) Nature Neuroscience 3, 757-8
25 Levin, B. E. and Dunn-Meynell, A. A. (1997) Brain Research 776, 146-53
26 Levin, B. E., Govek, E. K. and Dunn-Meynell, A. A. (1998) Brain Research 808, 317-9
27 Levin, B. E., Brown, K. L. and Dunn-Meynell, A. A. (1996) Brain Research 739, 293-300
28 Rowe, I. C., Boden, P. R. and Ashford, M. L. (1996) Journal of Physiology 497, 365-77
29 Fujimoto, K., Sakata, T., Arase, K., Kurata, K., Okabe, Y. and Shiraishi, T. (1985) Life Sciences 37, 2475-82

30 Kurata, K., Fujimoto, K. and Sakata, T. (1989) Metabolism: Clinical & Experimental 38, 46-51
31 Kurata, K., Fujimoto, K., Sakata, T., Etou, H. and Fukagawa, K. (1986) Physiology & Behavior 37, 615-20

## Claims

**1.** A compound of Formula (I):

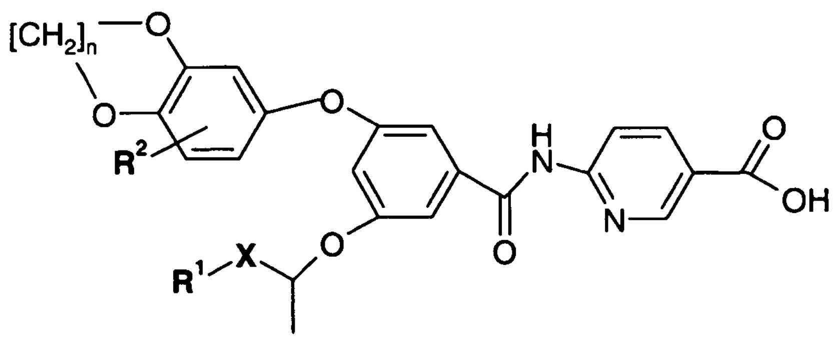

Formula (I)

wherein:

**$R^1$-X-** is selected from: methyl, methoxymethyl and

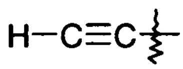

;

**$R^2$** is selected from hydrogen, methyl, chloro and fluoro;
**n** is 1 or 2;

or a salt, in-vivo hydrolysable ester or solvate thereof.

**2.** A compound as claimed in claim 1 of Formula (Ia)

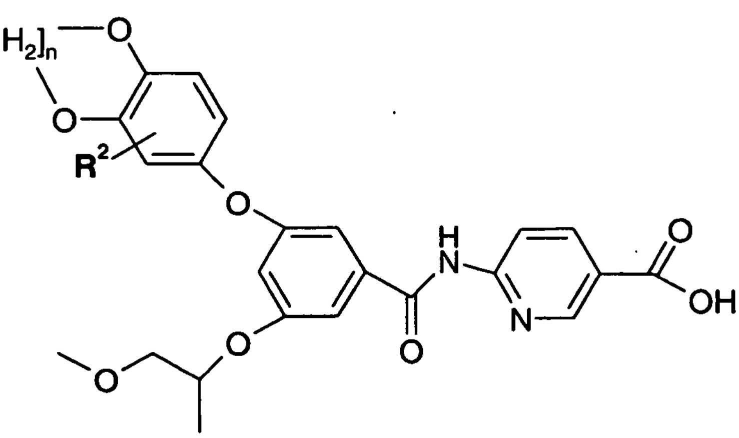

Formula (Ia)

wherein:

**n** and $\mathbf{R^2}$ are as defined above in a compound of Formula (I);

or a salt, solvate or in-vivo hydrolysable ester thereof.

**3.** A compound as claimed in claim 1 of Formula (Ib)

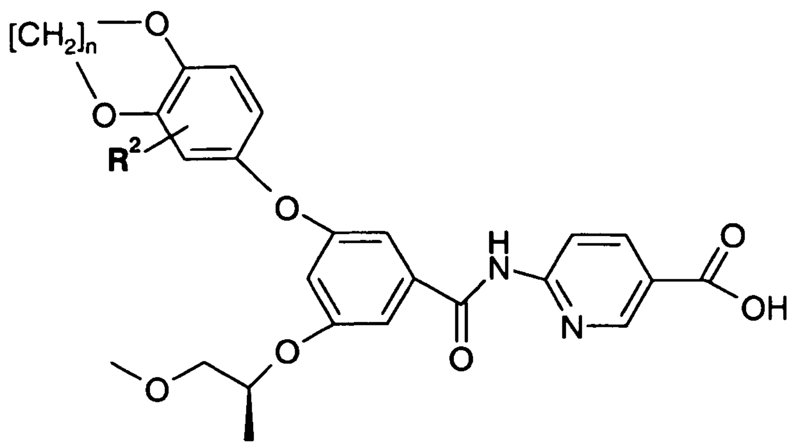

Formula (Ib)

wherein:

**n** and $\mathbf{R^2}$ are as defined above in a compound of Formula (I);

or a salt, solvate or in-vivo hydrolysable ester thereof.

**4.** A compound as claimed in claim 1 of Formula (Ic)

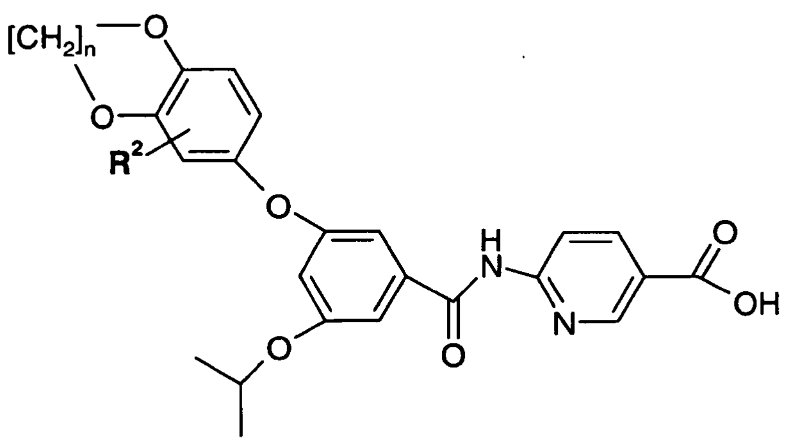

Formula (Ic)

wherein:

**n** and $\mathbf{R^2}$ are as defined above in a compound of Formula (I);

or a salt, solvate or in-vivo hydrolysable ester thereof.

5. A compound as claimed in claim 1 of Formula (Id)

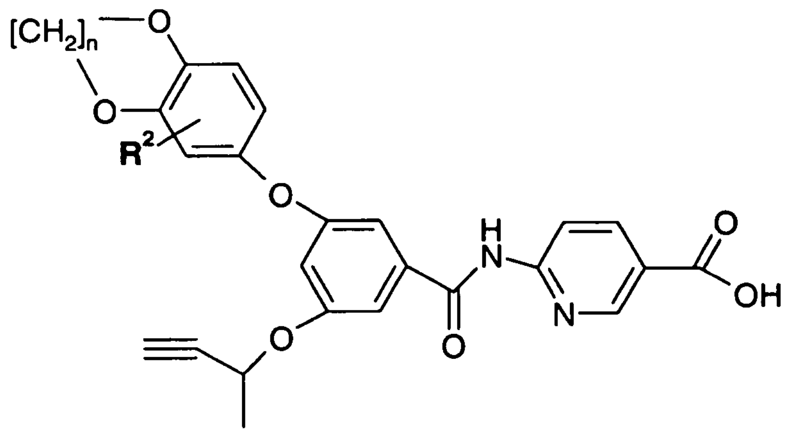

Formula (Id) ·

wherein:

**n** and $\mathbf{R^2}$ are as defined above in a compound of Formula (I);

or a salt, solvate or in-vivo hydrolysable ester thereof.

6. A compound as claimed in claim 1 of Formula (Ie)

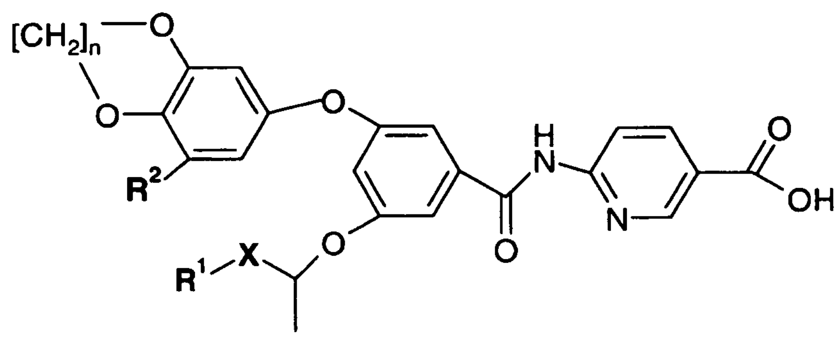

Formula (Ie)

wherein:

**n, X,** $\mathbf{R^1}$ and $\mathbf{R^2}$ are as defined above in a compound of Formula (I);

or a salt, solvate or in-vivo hydrolysable ester thereof.

7. A compound selected from one or more of the following:

6-{[(3-(2,3-dihydro-1,4-benzodioxin-6-yloxy)-5-{[(1S)-1-methyl-2-(methyloxy)ethyl]oxy}phenyl)carbonyl]amino}pyridine-3-carboxylic acid and
6- {[(3-(1,3-benzodioxol-5-yloxy)-5-{[(1S)-1-methyl-2-(methyloxy)ethyl]-oxy}phenyl)carbonyl]amino}pyridine-3-carboxylic acid

or a salt, solvate or in-vivo hydrolysable ester thereof.

**8.** A pharmaceutical composition comprising a compound of Formula (I) as claimed in any one of Claims 1 to 7, or a salt, solvate or in-vivo hydrolysable ester thereof, together with a pharmaceutically-acceptable diluent or carrier.

**9.** A compound of Formula (I), as claimed in any one of Claims 1 to 7, or a salt, solvate or in-vivo hydrolysable ester thereof, for use as a medicament.

**10.** A compound of Formula (I), as claimed in any one of Claims 1 to 7, or a salt, solvate or in-vivo hydrolysable ester thereof, for use in the preparation of a medicament for treatment of a disease mediated through GLK, in particular type 2 diabetes.

**11.** The use of a compound of Formula (I), as claimed in any one of Claims 1 to 7, or salt, solvate or in-vivo hydrolysable ester thereof, in the preparation of a medicament for use in the combined treatment or prevention of diabetes and obesity.

**12.** The use of a compound of Formula (I), as claimed in any one of Claims 1 to 7, or salt, solvate or in-vivo hydrolysable ester thereof, in the preparation of a medicament for use in the treatment or prevention of obesity.

**13.** A process for the preparation of a compound of Formula (I) as claimed in Claim 1, a salt, in-vivo hydrolysable ester or solvate thereof which comprises:

(a) reaction of an acid of Formula (IIIa) or activated derivative thereof with a compound of Formula (IIIb),

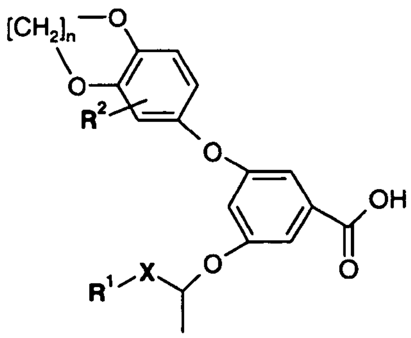

Formula (IIIa)

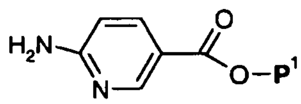

Formula (IIIb);

wherein **$P^1$** is hydrogen or a protecting group
or
(b) de-protection of a compound of Formula (IIIc),

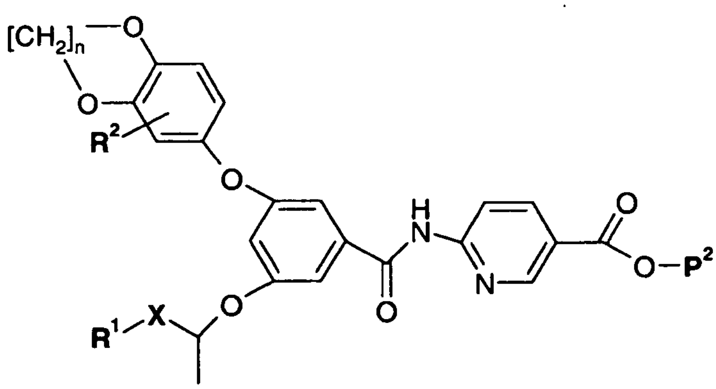

Formula (IIIc)

wherein $P^2$ is a protecting group; or
(c) reaction of a compound of Formula (IIId) with a compound of Formula (IIIe),

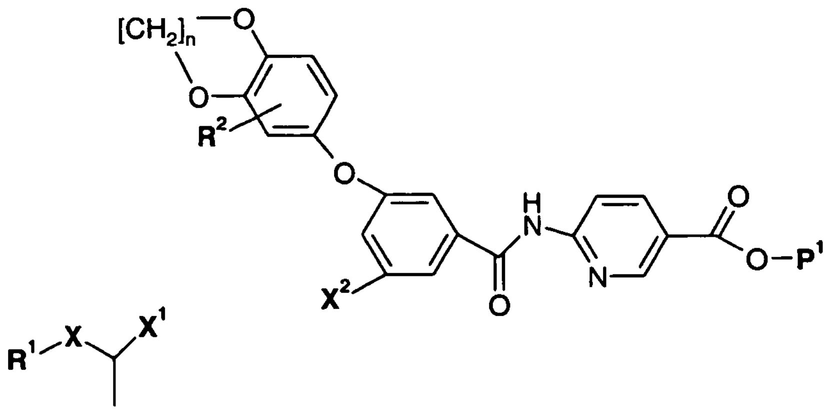

Formula (IIId) Formula (IIIe)

wherein $X^1$ is a leaving group and $X^2$ is a hydroxyl group or $X^1$ is a hydroxyl group and $X^2$ is a leaving group and wherein $P^1$ is hydrogen or a protecting group; or
(d) reaction of a compound of Formula (IIIf) with a compound of Formula (IIIg)

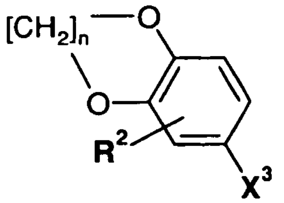

Formula (IIIf)

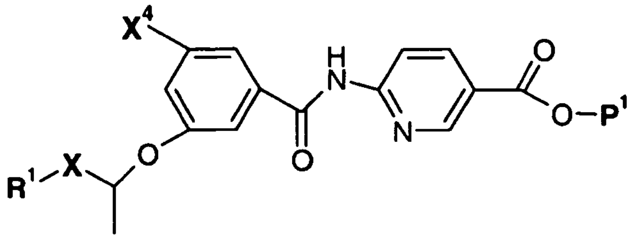

Formula (IIIg)

wherein $X^3$ is a leaving group or an organometallic reagent and $X^4$ is a hydroxyl group or $X^3$ is a hydroxyl group and $X^4$ is a leaving group or an organometallic reagent wherein $P^1$ is hydrogen or a protecting group; or
(e) reaction of a compound of Formula (IIIh) with a compound of Formula (IIIi),

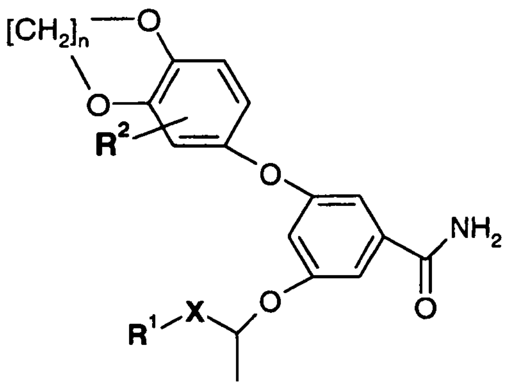

Formula (IIIh)

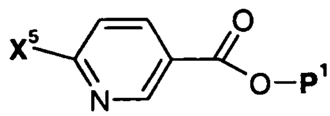

Formula (IIIi);

wherein $X^5$ is a leaving group and wherein $P^1$ is hydrogen or a protecting group; and thereafter, if necessary:

i) converting a compound of Formula (I) into another compound of Formula (I);
ii) removing any protecting groups;
iii) forming a salt, in-vivo hydrolysable ester or solvate thereof.

## Patentansprüche

**1.** Verbindungen der Formel (I):

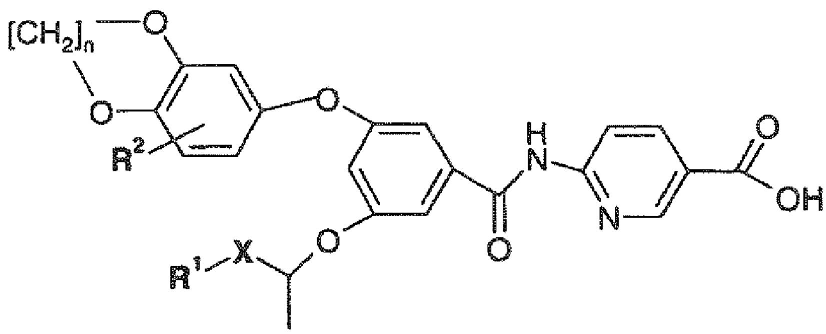

Formel (I)

in welcher:

**$R^1$-X-** ausgewählt ist aus: Methyl, Methoxymethyl und

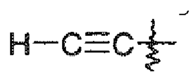
;

**$R^2$** ausgewählt ist aus Wasserstoff, Methyl, Chlor und Fluor;
**n** für 1 oder 2 steht;

und deren Salze, in vivo hydrolysierbare Ester und Solvate.

**2.** Verbindungen nach Anspruch 1 der Formel (Ia)

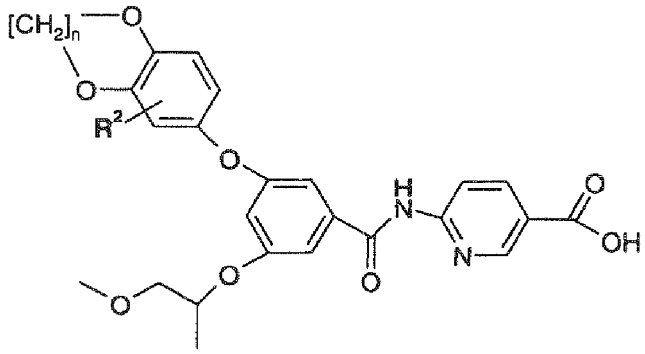

Formel (Ia)

in welcher:

**n** und $R^2$ wie oben für eine Verbindung der Formel (I) definiert sind;

und deren Salze, Solvate und in vivo hydrolysierbare Ester.

**3.** Verbindungen nach Anspruch 1 der Formel (Ib)

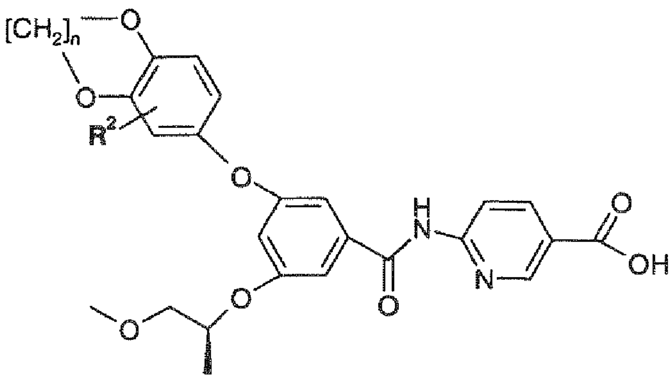

Formel (Ib)

in welcher:

**n** und $R^2$ wie oben für eine Verbindung der Formel (I) definiert sind;

und deren Salze, Solvate und in vivo hydrolysierbare Ester.

**4.** Verbindungen nach Anspruch 1 der Formel (Ic)

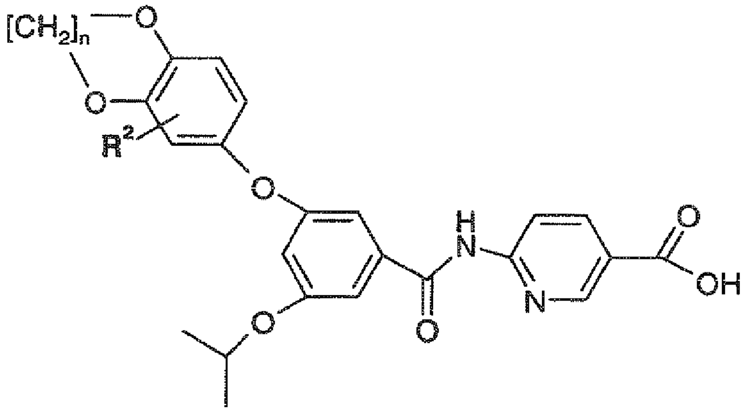

Formel (Ic)

in welcher:

**n** und $R^2$ wie oben für eine Verbindung der Formel (I) definiert sind;

und deren Salze, Solvate und in vivo hydrolysierbare Ester.

**5.** Verbindungen nach Anspruch 1 der Formel (Id)

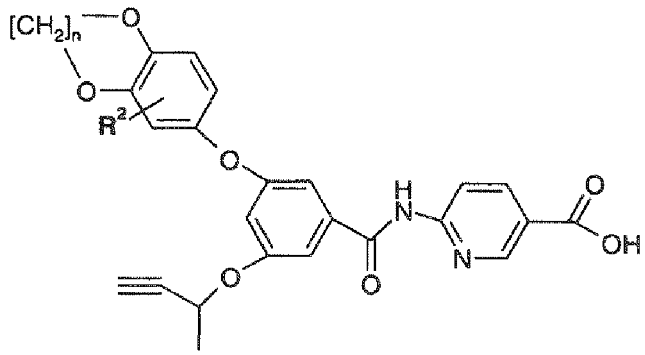

Formel (Id)

in welcher:

**n** und $R^2$ wie oben für eine Verbindung der Formel (I) definiert sind;

und deren Salze, Solvate und in vivo hydrolysierbare Ester.

**6.** Verbindungen nach Anspruch 1 der Formel (Ie)

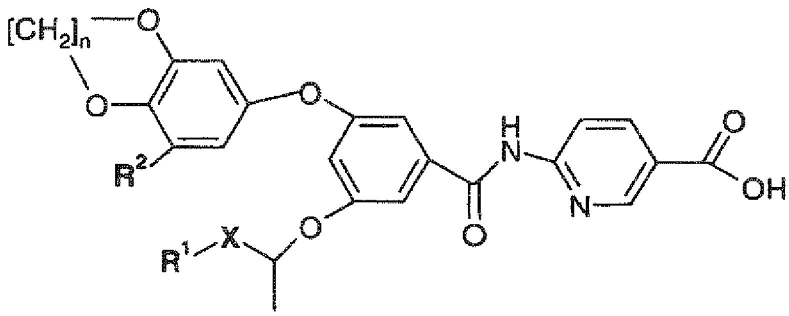

Formel (Ie)

in welcher:

**n, X, $R^1$** und **$R^2$** wie oben für eine Verbindung der Formel (I) definiert sind;

und deren Salze, Solvate und in vivo hydrolysierbare Ester.

**7.** Verbindungen, ausgewählt aus einer oder mehreren der folgenden:

6-{[3-(2,3-Dihydro-1,4-benzodioxin-6-yloxy)-5-{[(1S)-1-methyl-2-(methyloxy)ethyl]oxy}phenyl) carbonyl] amino}pyridin-3-carbonsäure und
6-{[(3-(1,3-Benzodioxol-5-yloxy)-5-{[(1S)-1-methyl-2-(methyloxy)ethyl]oxy}phenyl)carbonyl] amino}pyridin-3-carbonsäure

und deren Salzen, Solvaten und in vivo hydrolysierbaren Estern.

**8.** Pharmazeutische Zusammensetzung, enthaltend eine Verbindung der Formel (I) nach einem der Ansprüche 1 bis 7 oder ein Salz, ein Solvat oder einen in vivo hydrolysierbaren Ester davon zusammen mit einem pharmazeutisch annehmbaren Verdünnungsmittel oder Träger.

**9.** Verbindungen der Formel (I) nach einem der Ansprüche 1 bis 7 und deren Salze, Solvate und in vivo hydrolysierbare Ester zur Verwendung als Medikament.

**10.** Verbindungen der Formel (I) nach einem der Ansprüche 1 bis 7 und deren Salze, Solvate und in vivo hydrolysierbare Ester zur Verwendung bei der Herstellung eines Medikaments zur Behandlung einer durch GLK vermittelten Krankheit, insbesondere Typ-2-Diabetes.

**11.** Verwendung einer Verbindung der Formel (I) nach einem der Ansprüche 1 bis 7 oder eines Salzes, eines Solvates oder eines in vivo hydrolysierbaren Esters davon bei der Herstellung eines Medikaments zur Verwendung bei der kombinierten Behandlung oder Prävention von Diabetes und Obesitas.

**12.** Verwendung einer Verbindung der Formel (I) nach einem der Ansprüche 1 bis 7 oder eines Salzes, eines Solvates oder eines in vivo hydrolysierbaren Esters davon bei der Herstellung eines Medikaments zur Verwendung bei der Behandlung oder Prävention von Obesitas.

**13.** Verfahren zur Herstellung einer Verbindung der Formel (I) nach Anspruch 1 oder eines Salzes, eines in vivo hydrolysierbaren Esters oder eines Solvates davon, bei dem man:

(a) eine Säure der Formel (IIIa) oder ein aktiviertes Derivat davon mit einer Verbindung der Formel (IIIb)

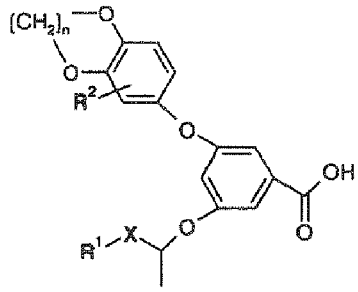

Formel (IIIa)

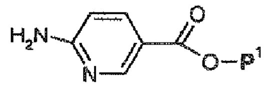

Formel (IIIb);

wobei **P¹** für Wasserstoff oder eine Schutzgruppe steht, umsetzt

oder

(b) eine Verbindung der Formel (IIIc)

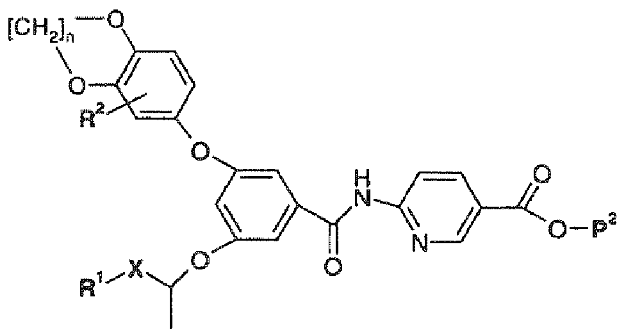

Formel (IIIc)

wobei **P²** für eine Schutzgruppe steht, entschützt;

oder

(c) eine Verbindung der Formel (IIId) mit einer Verbindung der Formel (IIIe)

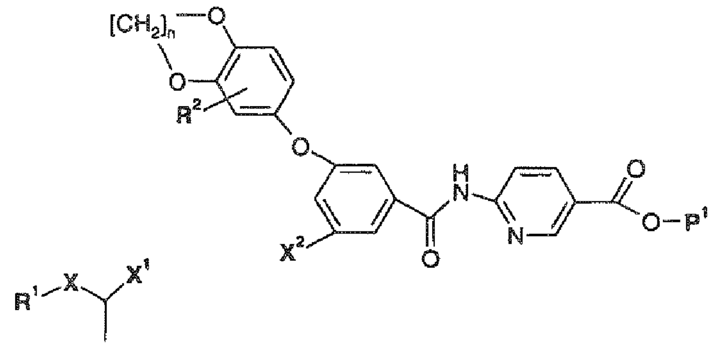

Formel (IIId) Formel (IIIe)

wobei $X^1$ für eine Abgangsgruppe steht und $X^2$ für eine Hydroxylgruppe steht oder $X^1$ für eine Hydroxylgruppe steht und $X^2$ für eine Abgangsgruppe steht und wobei $P^1$ für Wasserstoff oder eine Schutzgruppe steht, umsetzt; oder

(d) eine Verbindung der Formel (IIIf) mit einer Verbindung der Formel (IIIg)

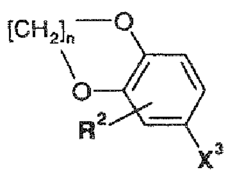

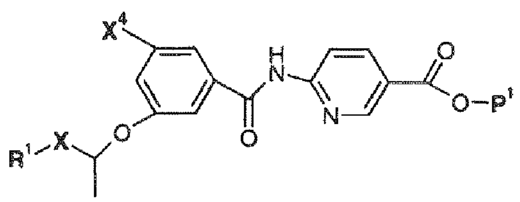

Formel (IIIf) Formel (IIIg)

wobei $X^3$ für eine Abgangsgruppe oder ein metallorganisches Reagens und $X^4$ für eine Hydroxylgruppe oder $X^3$ für eine Hydroxylgruppe und $X^4$ für eine Abgangsgruppe oder ein metallorganisches Reagens steht, wobei $P^1$ für Wasserstoff oder eine Schutzgruppe steht, umsetzt; oder

(e) eine Verbindung der Formel (IIIh) mit einer Verbindung der Formel (IIIi)

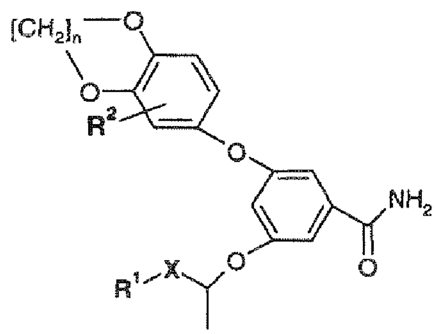

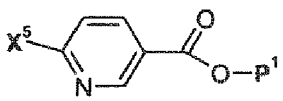

Formel (IIIh) Formel (IIIi);

wobei $X^5$ für eine Abgangsgruppe steht und wobei $P^1$ für Wasserstoff oder eine Schutzgruppe steht, umsetzt; und anschließend, falls erforderlich:

i) eine Verbindung der Formel (I) in eine andere Verbindung der Formel (I) umwandelt;

ii) gegebenenfalls vorhandene Schutzgruppen entfernt;
iii) ein Salz, einen in vivo hydrolysierbaren Ester oder ein Solvat davon bildet.

## Revendications

**1.** Composé de formule (I) :

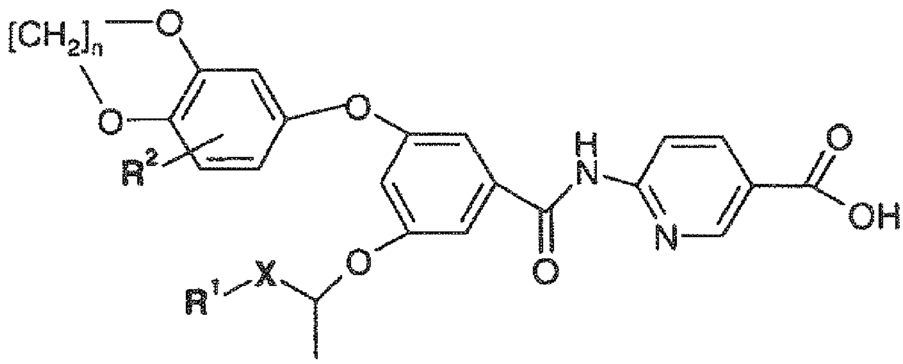

Formule (I)

dans laquelle :

**$R^1$-X-** est choisi parmi : méthyle, méthoxyméthyle et

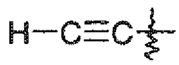

;

**$R^2$** est choisi parmi hydrogène, méthyle, chloro et fluoro ;
**n** est 1 ou 2 ;

ou un sel, un ester hydrolysable in vivo ou un solvate de celui-ci.

**2.** Composé selon la revendication 1, de formule (Ia)

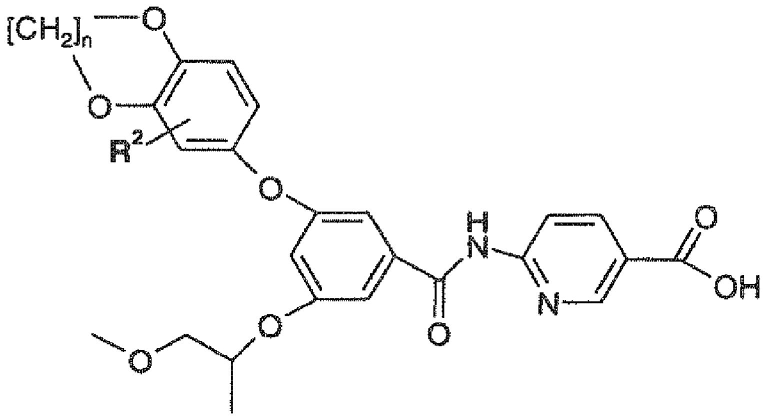

Formule (Ia)

dans laquelle :

**n** et $\mathbf{R^2}$ sont tels que définis ci-dessus dans un composé de formule (I) ;

ou un sel, un solvate ou un ester hydrolysable in vivo de celui-ci.

**3.** Composé selon la revendication 1, de formule (Ib)

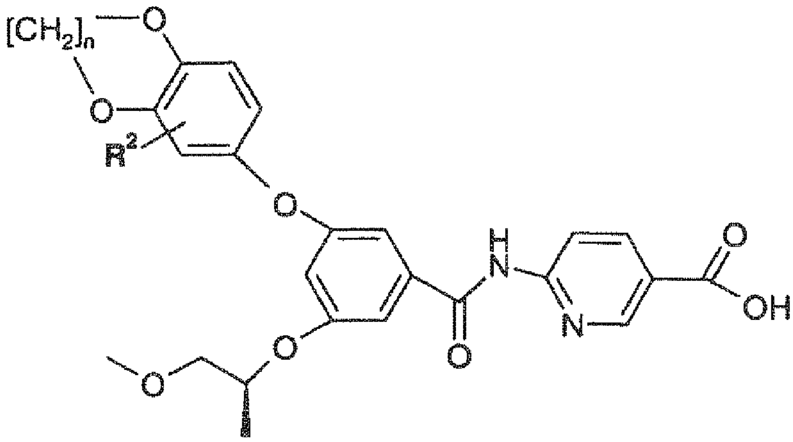

Formule (Ib)

dans laquelle :

**n** et $\mathbf{R^2}$ sont tels que définis ci-dessus dans un composé de formule (I) ;

ou un sel, un solvate ou un ester hydrolysable in vivo de celui-ci.

**4.** Composé selon la revendication 1, de formule (Ic)

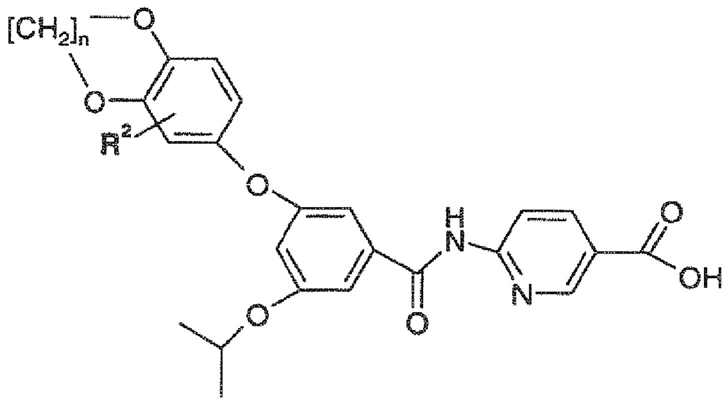

Formule (Ic)

dans laquelle :

**n** et $\mathbf{R^2}$ sont tels que définis ci-dessus dans un composé de formule (I) ;

ou un sel, un solvate ou un ester hydrolysable in vivo de celui-ci.

**5.** Composé selon la revendication 1, de formule (Id)

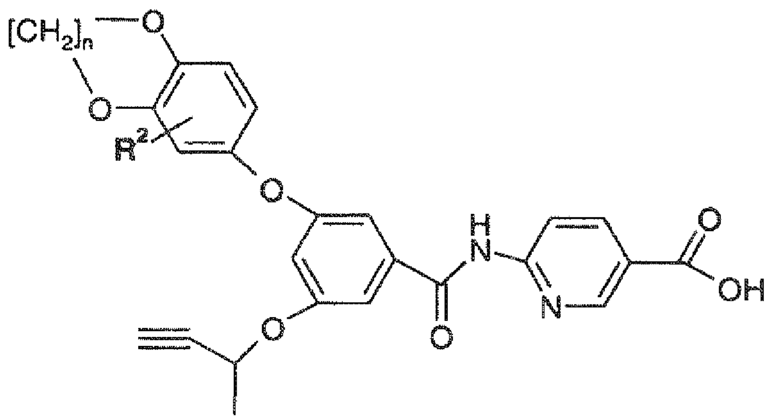

Formule (Id)

dans laquelle :

**n** et $\mathbf{R^2}$ sont tels que définis ci-dessus dans un composé de formule (I) ;

ou un sel, un solvate ou un ester hydrolysable in vivo de celui-ci.

**6.** Composé selon la revendication 1, de formule (Ie)

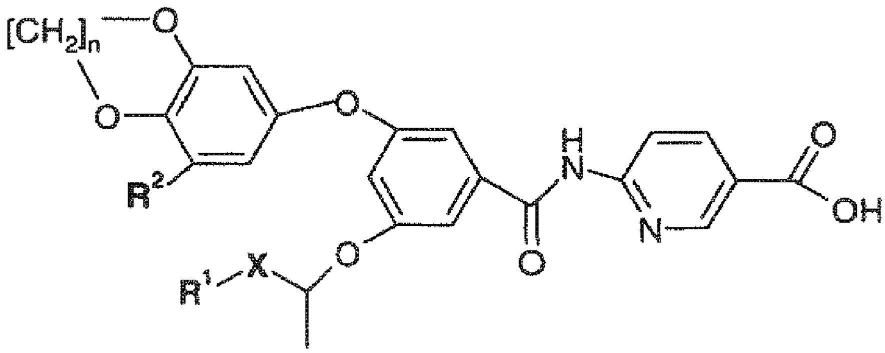

Formule (Ie)

dans laquelle :

**n, X, $R^1$** et **$R^2$** sont tels que définis ci-dessus dans un composé de formule (I) ;

ou un sel, un solvate ou un ester hydrolysable in vivo de celui-ci.

**7.** Composé choisi parmi un ou plusieurs de ce qui suit :

l'acide 6-{[(3-(2,3-dihydro-1,4-benzodioxin-6-yloxy)-5-{[(1S)-1-méthyl-2-(méthyloxy)éthyl]oxy}phényl)carbonyl] amino} pyridine-3-carboxylique et
l'acide 6-{[(3-(1,3-benzodioxol-5-yloxy)-5-{[(1S)-1-méthyl-2-(méthyloxy)éthyl]oxy}phényl)carbonyl] amino}pyridine-3-carboxylique

ou un sel, un solvate ou un ester hydrolysable in vivo de celui-ci.

**8.** Composition pharmaceutique comprenant un composé de formule (I) selon l'une quelconque des revendications 1 à 7, ou un sel, un solvate ou un ester hydrolysable in vivo de celui-ci, conjointement avec un diluant ou un support pharmaceutiquement acceptable.

**9.** Composé de formule (I) selon l'une quelconque des revendications 1 à 7, ou un sel, un solvate ou un ester hydrolysable in vivo de celui-ci, destiné à être utilisé comme médicament.

**10.** Composé de formule (I) selon l'une quelconque des revendications 1 à 7, ou un sel, un solvate ou un ester hydrolysable in vivo de celui-ci, destiné à être utilisé dans la préparation d'un médicament destiné au traitement d'une maladie médiée par la GLK, en particulier du diabète de type 2.

**11.** Utilisation d'un composé de formule (I) selon l'une quelconque des revendications 1 à 7, ou d'un sel, d'un solvate ou d'un ester hydrolysable in vivo de celui-ci, dans la préparation d'un médicament destiné à être utilisé dans le traitement ou la prévention combiné du diabète et de l'obésité.

**12.** Utilisation d'un composé de formule (I) selon l'une quelconque des revendications 1 à 7, ou d'un sel, d'un solvate ou d'un ester hydrolysable in vivo de celui-ci, dans la préparation d'un médicament destiné à être utilisé dans le traitement ou la prévention de l'obésité.

**13.** Procédé de préparation d'un composé de formule (I) selon la revendication 1, d'un sel, d'un ester hydrolysable in vivo ou d'un solvate de celui-ci, qui comprend :

(a) la réaction d'un acide de formule (IIIa) ou d'un dérivé activé de celui-ci avec un composé de formule (IIIb)

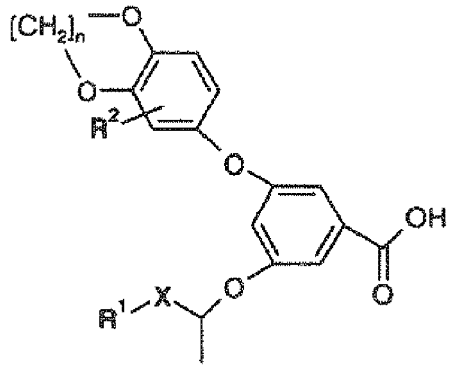

Formule (IIIa)

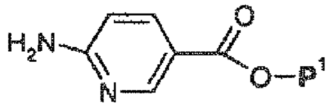

Formule (IIIb);

dans lesquelles $P^1$ est hydrogène ou un groupement protecteur
ou
(b) la déprotection d'un composé de formule (IIIc)

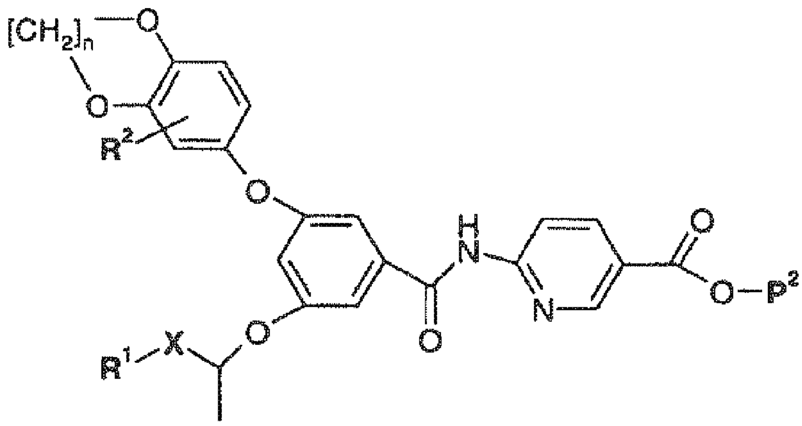

Formule (IIIc)

dans laquelle $P^2$ est un groupement protecteur ; ou
(c) la réaction d'un composé de formule (IIId) avec un composé de formule (IIIe),

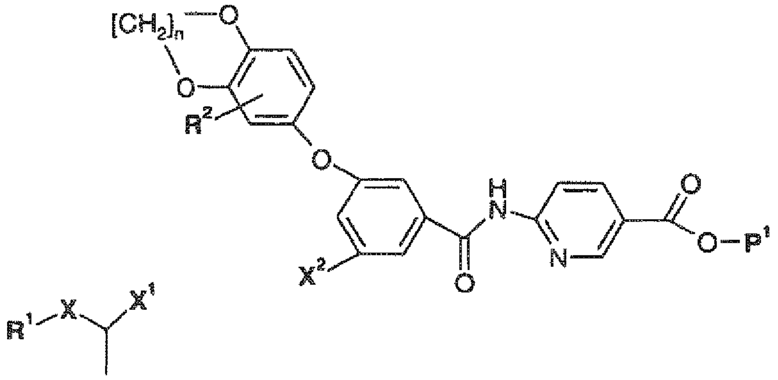

Formule (IIId) Formule (IIIe)

dans lesquelles **$X^1$** est un groupement partant et **$X^2$** est un groupement hydroxy ou **$X^1$** est un groupement hydroxy et **$X^2$** est un groupement partant et dans lesquelles **$P^1$** est hydrogène ou un groupement protecteur ; ou

(d) la réaction d'un composé de formule (IIIf) avec un composé de formule (IIIg)

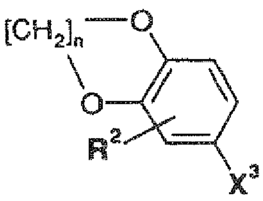

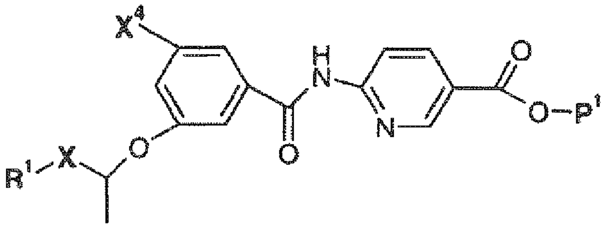

Formule (IIIf) Formule (IIIg)

dans lesquelles **$X^3$** est un groupement partant ou un réactif organométallique et **$X^4$** est un groupement hydroxy ou **$X^3$** est un groupement hydroxy et **$X^4$** est un groupement partant ou un réactif organométallique et dans lesquelles **$P^1$** est hydrogène ou un groupement protecteur ; ou

(e) la réaction d'un composé de formule (IIIh) avec un composé de formule (IIIi),

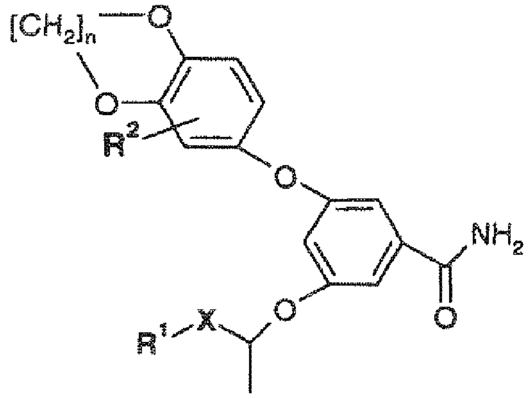

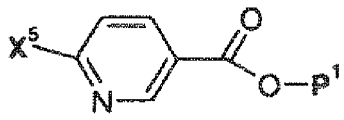

Formule (IIIh) Formule (IIIi);

dans lesquelles **$X^5$** est un groupement partant et dans lesquelles **$P^1$** est hydrogène ou un groupement protecteur ; et ensuite, le cas échéant :

i) la transformation d'un composé de formule (I) en un autre composé de formule (I) ;
ii) l'élimination de tout groupement protecteur ;
iii) la formation d'un sel, d'un ester hydrolysable in vivo ou d'un solvate de celui-ci.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0058293 A
- WO 0144216 A, Roche
- WO 9622282 A
- WO 9622293 A
- WO 9622294 A
- WO 9622295 A
- WO 9749707 A
- WO 9749708 A
- WO 9641795 A
- JP 8143565 B
- JP 8301760 B
- EP 619116 A
- WO 0112621 A
- WO 0026202 A
- GB 2331748 A
- WO 9636619 A
- US 5466715 A
- US 5258407 A
- JP 58069812 B
- US 3950351 A
- WO 03000262 A
- WO 03015774 A
- WO 03066613 A
- GB 0202873 W
- WO 03000267 A
- WO 0120327 A


### Non-patent literature cited in the description


- **CUSHMAN et al.** *Bioorg Med Chem Lett,* 1991, vol. 1 (4), 211-14
- **ROGERS et al.** *J Med Chem,* 1981, vol. 24 (11), 1284-7
- **CAVIER et al.** *Eur J Med Chem - Chim Ther,* 1978, vol. 13 (6), 539-43
- Design of Prodrugs. Elsevier, 1985
- Methods in Enzymology. Academic Press, 1985, vol. 42, 309-396
- A Textbook of Drug Design and Development
- **H. BUNDGAARD.** Design and Application of Prodrugs. 1991, 113-191
- **H. BUNDGAARD.** *Advanced Drug Delivery Reviews,* 1992, vol. 8, 1-38
- **H. BUNDGAARD et al.** *Journal of Pharmaceutical Sciences,* 1988, vol. 77, 285
- **N. KAKEYA et al.** *Chem Pharm Bull,* 1984, vol. 32, 692
- Chairman of Editorial Board. **CORWIN HANSCH.** Comprehensive Medicinal Chemistry. Pergamon Press, 1990, vol. 5
- **T.W. GREENE ; P.G.M. WUTS.** Protective Groups in Organic Synthesis. John Wiley & Sons, 1991
- **BROCLDEHURST et al.** *Diabetes,* 2004, vol. 53, 535-541
- **W. LINDNER et al.** *J.Chromatography,* 1996, vol. 677, 1-28
- **PRINTZ, R. L. ; MAGNUSON, M. A. ; GRANNER, D. K.** *Annual Review of Nutrition,* 1993, vol. 13, 463-96
- **DEFRONZO, R. A.** *Diabetes,* 1988, vol. 37, 667-87
- **FROGUEL, P. ; ZOUALI, H. ; VIONNET, N. ; VELHO, G. ; VAXILLAIRE, M. ; SUN, F. ; LESAGE, S. ; STOFFEL, M. ; TAKEDA, J. ; PASSA, P.** *New England Journal of Medicine,* 1993, vol. 328, 697-702
- **BELL, G. I. ; PILKIS, S. J. ; WEBER, I. T. ; POLONSKY, K. S.** *Annual Review of Physiology,* 1996, vol. 58, 171-86
- **VELHO, G. ; PETERSEN, K. F. ; PERSEGHIN, G. ; HWANG, J. H. ; ROTHMAN, D. L. ; PUEYO, M. E. ; CLINE, G. W. ; FROGUEL, P. ; SHULMAN, G. I.** *Journal of Clinical Investigation,* 1996, vol. 98, 1755-61
- **GLOYN, A.L. ; NOORDAM, K. ; WILLEMSEN, M.A.A.P. ; ELLARD, S. ; LAM, W.W.K. ; CAMPBELL, I. W. ; MIDGLEY, P. ; SHIOTA, C. ; BUETTGER, C. ; MAGNUSON, M.A.** *Diabetes,* vol. 52, 2433-2440
- **CHRISTESEN, H. B. ; JACOBSEN, B. B. ; ODILI, S. ; BUETTGER, C. ; CUESTA-MUNOZ, A. ; HANSEN, T. ; BRUSGAARD, K. ; MASSA, O. ; MAGNUSON, M. A. ; SHIOTA, C.** *Diabetes,* 2002, vol. 51, 1240-6
- **GLASER, B. ; KESAVAN, P. ; HEYMAN, M. ; DAVIS, E. ; CUESTA, A. ; BUCHS, A. ; STANLEY, C. A. ; THORNTON, P. S. ; PERMUTT, M. A. ; MATSCHINSKY, F. M.** *New England Journal of Medicine,* 1998, vol. 338, 226-30

- **CARO, J. F. ; TRIESTER, S. ; PATEL, V. K. ; TAPSCOTT, E. B. ; FRAZIER, N. L. ; DOHM, G. L.** *Hormone & Metabolic Research,* 1995, vol. 27, 19-22
- **DESAI, U. J. ; SLOSBERG, E. D. ; BOETTCHER, B. R. ; CAPLAN, S. L. ; FANELLI, B. ; STEPHAN, Z. ; GUNTHER, V. J. ; KALEKO, M. ; CONNELLY, S.** *Diabetes,* 2001, vol. 50, 2287-95
- **SHIOTA, M. ; POSTIC, C. ; FUJIMOTO, Y. ; JETTON, T. L. ; DIXON, K. ; PAN, D. ; GRIMSBY, J. ; GRIPPO, J. F. ; MAGNUSON, M. A. ; CHERRINGTON, A. D.** *Diabetes,* 2001, vol. 50, 622-9
- **FERRE, T. ; PUJOL, A. ; RIU, E. ; BOSCH, F. ; VALERA, A.** *Proceedings of the National Academy of Sciences of the United States of America,* 1996, vol. 93, 7225-30
- **SEOANE, J. ; BARBERA, A. ; TELEMAQUE-POTTS, S. ; NEWGARD, C. B. ; GUINOVART, J. J.** *Journal of Biological Chemistry,* 1999, vol. 274, 31833-8
- **MOORE, M. C. ; DAVIS, S. N. ; MANN, S. L. ; CHERRINGTON, A. D.** *Diabetes Care,* 2001, vol. 24, 1882-7
- **ALVAREZ, E. ; RONCERO, I. ; CHOWEN, J. A. ; VAZQUEZ, P. ; BLAZQUEZ, E.** *Journal of Neurochemistry,* 2002, vol. 80, 45-53
- **LYNCH, R. M. ; TOMPKINS, L. S. ; BROOKS, H. L. ; DUNN-MEYNELL, A. A. ; LEVIN, B. E.** *Diabetes,* 2000, vol. 49, 693-700
- **RONCERO, I. ; ALVAREZ, E. ; VAZQUEZ, P. ; BLAZQUEZ, E.** *Journal of Neurochemistry,* 2000, vol. 74, 1848-57
- **YANG, X. J. ; KOW, L. M. ; FUNABASHI, T. ; MOBBS, C. V.** *Diabetes,* 1999, vol. 48, 1763-1772
- **SCHUIT, F. C. ; HUYPENS, P. ; HEIMBERG, H. ; PIPELEERS, D. G.** *Diabetes,* 2001, vol. 50, 1-11
- **LEVIN, B. E.** *International Journal of Obesity,* 2001, vol. 25 (5), S68-S72
- **ALVAREZ, E. ; RONCERO, I. ; CHOWEN, J. A. ; THORENS, B. ; BLAZQUEZ, E.** *Journal of Neurochemistry,* 1996, vol. 66, 920-7
- **MOBBS, C. V. ; KOW, L. M. ; YANG, X. J.** *American Journal of Physiology - Endocrinology & Metabolism,* 2001, vol. 281, E649-54
- **LEVIN, B. E. ; DUNN-MEYNELL, A. A. ; ROUTH, V. H.** *American Journal of Physiology,* 1999, vol. 276, R1223-31
- **SPANSWICK, D. ; SMITH, M. A. ; GROPPI, V. E. ; LOGAN, S. D. ; ASHFORD, M. L.** *Nature,* 1997, vol. 390, 521-5
- **SPANSWICK, D. ; SMITH, M. A. ; MIRSHAMSI, S. ; ROUTH, V. H. ; ASHFORD, M. L.** *Nature Neuroscience,* 2000, vol. 3, 757-8
- **LEVIN, B. E. ; DUNN-MEYNELL, A. A.** *Brain Research,* 1997, vol. 776, 146-53
- **LEVIN, B. E. ; GOVEK, E. K. ; DUNN-MEYNELL, A. A.** *Brain Research,* 1998, vol. 808, 317-9
- **LEVIN, B. E. ; BROWN, K. L. ; DUNN-MEYNELL, A. A.** *Brain Research,* 1996, vol. 739, 293-300
- **ROWE, I. C. ; BODEN, P. R. ; ASHFORD, M. L.** *Journal of Physiology,* 1996, vol. 497, 365-77
- **FUJIMOTO, K. ; SAKATA, T. ; ARASE, K. ; KURATA, K. ; OKABE, Y. ; SHIRAISHI, T.** *Life Sciences,* 1985, vol. 37, 2475-82
- **KURATA, K. ; FUJIMOTO, K. ; SAKATA, T.** *Metabolism: Clinical & Experimental,* 1989, vol. 38, 46-51
- **KURATA, K. ; FUJIMOTO, K. ; SAKATA, T. ; ETOU, H. ; FUKAGAWA, K.** *Physiology & Behavior,* 1986, vol. 37, 615-20